(19) Europäisches Patentamt
European Patent Office
Office européen des brevets

(11) Publication number : **0 468 714 A2**

(12) # EUROPEAN PATENT APPLICATION

(21) Application number : **91306620.5**

(22) Date of filing : **19.07.91**

(51) Int. Cl.[5] : **C07K 7/06,** C07K 7/08, C07K 7/54, C07K 15/04, C07K 17/10, A61K 39/21

(30) Priority : **19.07.90 US 555558**
**19.06.91 US 715275**
**19.07.90 US 555974**
**19.06.91 US 715277**

(43) Date of publication of application :
**29.01.92 Bulletin 92/05**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Marburg, Stephen**
**50 Concord Avenue**
**Metuchen, NJ 08840 (US)**
Inventor : **Tolman, Richard L.**
**29 Upper Warren Way**
**Warren, NJ 07059 (US)**
Inventor : **Emini, Emilio A.**
**6 Fagge Manor Lane**
**Paoli, PA 19301 (US)**

(74) Representative : **Barrett-Major, Julie Diane et al**
**Merck & Co., Inc. European Patent Department Terlings Park Eastwick Road**
**Harlow Essex CM20 2QR (GB)**

(54) **Peptide-polysaccharide-protein conjugate vaccines.**

(57) Covalent conjugate immunogens, having Human Immunodeficiency Virus (HIV) Principal Neutralizing Determinant (PND) peptides covalently bound to the Outer Membrane Protein Complex (OMPC) of Neisseria through a linker which is an anionic polysaccharide (PSA), are useful for inducing anti-peptide immune responses in mammals, for inducing HIV-neutralizing antibodies in mammals, for formulating vaccines to prevent HIV infection or disease, including the Acquired Immune Deficiency Syndrome (AIDS) or for treating humans afflicted with HIV infection or HIV disease, including AIDS.

EP 0 468 714 A2

## BACKGROUND OF THE INVENTION

This invention is concerned with novel covalent peptide-polysaccharide-protein conjugates and a method of making and a method of using such conjugates. Principal Neutralizing Determinant (PND) peptides, having the property of high affinity binding to antibodies capable of neutralizing Human Immunodeficiency Virus (HIV), are conjugated to a carrier which is comprised of anionic polysaccharide (PSA) bound to an immunogenic protein or protein complex, such as the Outer Membrane Protein Complex (OMPC) of Neisseria.

The term "neutralizing" as applied to antibodies means that viral exposure to such antibodies, whether in vitro ou in vivo, results in the attenuation or abrogation of any or all of the recognized virus-mediated pathophysiologic functions characteristic of HIV infection and disease, such as Aquired Immune Deficiency Syndrome (AIDS) or AIDS Related Complex (ARC) including cellular fusion, cellular infection, CD4 receptor bearing cell depletion, and viral proliferation. Neutralizing antibodies meeting these criteria have been detected in the sera of HIV-infected patients and have been induced in animals and humans by immunization with diverse HIV related antigens.

Attenuated or killed whole HIV, HIV subunit proteins, live recombinant vaccinia virus containing incorporated HIV genetic material, and HIV specific peptides have all been evaluated for potential use as protective or therapeutic post-infection immunogens. The whole-virus approach is attended by the danger, however small, of producing an active infection in vaccine recipients, while the subunit-protein-vaccine approach has met with limited success in the induction of virus-neutraling antibodies. The live-recombinant-vaccinia-virus vaccine approach has promise, but the dangers inherent in the introduction of a live virus, however benign, especially into an already immunocompromised recipient, are obvious. Thus far, peptide based immunogens hold the most promise. The following references provide a general overview of ongoing vaccine evaluations: Lasky, L. A., Crit. Rev. in Immunol. 9, 153 (1989); Garrison, L., Clements, L. M., Comprehensive Therapy 15, 47 (1989); Dalgleish, A., Drugs of Today, 25, 709 (1989); Schulhafer, E. P., Verma, R. S., In Vivo 3, 61 (1989); Fauci, A. S., et al., Annals of Internal Medicine 110, 373 (1989); Rosenberg, Z. F., Fauci, A. S., Advances in Immunol. 47, 377 (1989); and Snart, R. S. AIDS 2, S107 (1988).

Peptides of interest in this invention, hereinafter referred to as Principal Neutralizing Determinants (PNDs), have been identified which are capable of eliciting HIV neutralizing immune responses in mammals. Although immunogenicity can be conferred on other HIV related or unrelated peptides upon conjugation according to this invention, of particular interest here is the PND located in the HIV IIIB and in most other HIV isolates, such as the MN isolate, at or near the amino acids between 296 and 341 of the HIV envelope glycoprotein, gp120 [numbering according to the scheme of Ratner et al., Nature 313, 277 (1985)]. Although the amino acid sequence in this region is variable across HIV isolates, the inter-isolate conserved core amino acid sequence, Gly-Pro-Gly (GPG), appears in over 90% of the isolates tested in one study, while the sequence Gly-Pro-Gly-Arg-Ala-Phe (GPGRAF) appears in a large number of common isolates [Goudsmit, J., Aids 2, S41 (1988)]. Less highly conserved amino acids appear on either side of the GPG trimer. A minimum of between 5 and 8 amino acids, including the GPG, appears to be necessary to induce an HIV neutralizing response [Javaherian et al., PNAS USA 86, 6768 (1989); Goudsmit et al., Res. Virol. 140, 419 (1989)].

Linear or cyclic peptides may be utilized to make conjugates which generate HIV neutralizing immune responses. Small divergences in amino acid sequence, for example the substitution of a valine for an alanine, or an aspartate for a glutamate, may in some cases give rise to peptides capable of eliciting similar immune responses. Furthermore, peptides having conserved tertiary structures but having divergent primary structures, as in a series of cyclic PND peptides (cPNDs), may give rise to similar immune responses.

Because HIV is known to be transmitted in either a cell-free or a cell-associated form, it may be an essential requirement that peptidyl epitopes be capable of priming both B-cell- and T-cell- mediated immune responses, such as antibody production and antibody-dependent cellular cytotoxicity, in order to be useful as anti-HIV immunogens. Peptide sequences from the gp120 region described above have been shown to be capable of inducing both types of immune responses [Goudsmit, J., AIDS 2, S41 (1988)].

In addition, in order to generate a useful anti-HIV vaccine, PND peptides, which are generally poorly immunogenic on their own, often must be conjugated to a carrier in a reproducible and quantifiable fashion. Unconjugated peptides are not only poor inducers of B-cell-mediated antibody production, they are also weak inducers of protective T-cell responses. The instant invention overcomes these problems by providing novel immunological conjugates of the PND peptides and immune enhancers.

In US Patent 4,695,624, Marburg et al. disclosed conjugation chemistry for covalently coupling the Haemophilus influenzae b capsular polysaccharide, polyribosyl ribitol phosphate (PRP) to the OMPC of Neisseria meningitidis. The conjugates of that invention elicited anti-PRP immune responses and were useful as immunogens to prevent Haemophilus influenzae b infections. The conjugates of this invention raise a completely different immune response against the HIV PND peptides which are absent in the conjugates of the

4,695,624 patent.

The novel conjugates of this invention are useful for inducing mammalian immune responses against the peptidyl portion of the conjugate. Where the peptide component of the conjugate represents an HIV PND peptide, or a peptide capable of eliciting immune responses which recognize HIV PND peptides, the conjugates are useful for inducing anti-HIV PND peptide antibodies in mammals, for inducing HIV-neutralizing antibodies in mammals, or for vaccinating humans prior to or after contraction of HIV infection or HIV disease, including AIDS.

## SUMMARY OF THE INVENTION

The conjugates of this invention are comprised of human immunodeficiency virus (HIV) Principal Neutralizing Determinant (PND) peptides covalently coupled to an immunogenic protein (PRO) through an anionic polysaccharide (PSA). A preferred embodiment of the invention may be represented by the structure IV:

$$\left[\left[PEP-r-\right]_{PF}PSA-B-\right]_{GF}PRO \qquad \underline{IV}$$

wherein:

- PEP represents covalently coupled HIV PND peptide, having either a linear or a cyclic structure;
- PSA represents anionic polysaccharide;
- PRO represents an immunogenic protein or protein complex, and preferably the outer membrane protein complex of Neisseria meningitidis;
- -r- represents a spacer forming a covalent linkage between the peptide and polysaccharide of the conjugate;
- -B- represents a spacer forming a covalent linkage between the polysaccharide and protein of the conjugate;
- PF = 0.001 to 10, and preferably about 0.01 to 1 milligrams PEP per milligram PSA;
- GF = 0.001 to 10, and preferably about 0.01 to 0.33 milligrams PSA per milligram PRO;
- ELF= epitopic loading factor, milligrams PEP per milligram PRO, and is preferably in the range 0.0001 to 0.5 mg PEP/mg PRO.

The conjugates of the invention are prepared by the process of covalently coupling separately-activated peptide, polysaccharide, and protein components to each other. The peptide and protein components are activated to display either pendant electrophilic or nucleophilic groups, and cannot react with each other. The polysaccharide component is conversely activated so as to display pendant nucleophilic or electrophilic groups, thus allowing reaction with the oppositely-activated peptides and proteins. Each reactive event results in the formation of a stable, covalent, bigeneric spacer that bonds either peptide or protein to the polysaccharide.

The covalent conjugate immunogens that result from this series of reactions may conveniently be thought of as a scaffold in which multiple peptide moieties are built upon a foundation of polysaccharide which is in turn anchored to protein. When the peptide components are immunologically equivalent to PNDs of HIV, conjugates produced by this process may be administred to mammals in immunologically effective amounts, with or without additional immunomodulatory, antiviral, or antibacterial compounds, and are useful for inducing mammalian immune responses against the peptidyl portion of the conjugates, For inducing HIV-neutralizing antibodies in mammals, or for making vaccines for administration to humans to prevent contraction of HIV infection or HIV disease, or for administration to humans afflicted with HIV infection or HIV disease, including AIDS.

## OBJECTS OF THE INVENTION:

Accordingly, it is an object of this invention to provide conjugates that are highly immunogenic and are capable of raising an immune response in mammals specific to the epitopes presented by the peptidyl portion of the conjugates. Another object is to provide a covalent conjugate immunogen wherein the peptide portions of the peptide-polysaccharide-protein conjugates are capable of eliciting mammalian immune responses which recognize HIV Principal Neutralizing Determinants. Another object is to provide a conjugate immunogen capable of raising mammalian antibody responses which neutralize the Human Immunodeficiency Virus. Another object is to provide a process for the high-yield production of water-soluble, covalent peptide-polysaccharide-protein conjugates. Another object is to provide a method of using such conjugate immunogens to raise anti-peptide, anti-HIV, or HIV-neutralizing immune responses in mammalian recipients. Another object is to use vaccine formulations containing the conjugate of this invention to immunize humans prior to or after contraction of HIV infection or HIV disease, including AIDS or ARC.

## DEFINITIONS AND ABBREVIATIONS

| | |
|---|---|
| AA assay | amino acid analysis method wherein peptides or proteins are acid hydrolyzed to the free amino acids and then quantitated |
| Acm | acetamidomethyl thiol protecting group |
| activation | reaction of peptides, proteins, or polysaccharide moieties with a reagent capable of derivatizing the moiety in order to enable subsequent desirable reactions to occur |
| AIDS | Acquired Immune Deficiency Syndrome |
| amino acid | a molecule having both an acid and amino functional group; there are 20 common α-amino acids with the general structure $H_2N$-CHR-COOH, wherein the R group defines the identity of the amino acid; these amino acids may have either a D or L stereochemical form and unless specified otherwise, by the lower case single letter abbreviation, or by the prefix "D-" before an amino acid name, the amino acid is of the natural or L configuration; the names of the 20 common amino acids and the structure of the R group are identified herein in single-letter code according to the following table: |

| AMINO ACID NAME | 3-letter code | 1-letter code | side-chain (R) |
|---|---|---|---|
| Alanine | Ala | A | $-CH_3$ |
| Arginine | Arg | R | $-(CH_2)_3NHCHNH_2NH_2^+$ |
| Asparagine | Asn | N | $-CH_2CONH_2$ |
| Aspartic Acid | Asp | D | $-CH_2COOH$ |
| Cysteine | Cys | C | $-CH_2SH$ |
| Glutamic Acid | Glu | E | $-(CH_2)_2COOH$ |
| Glutamine | Gln | Q | $-(CH_2)_2CONH_2$ |
| Glycine | Gly | G | -H |
| Histidine | His | H | $-CH_2$-imidazole |
| Isoleucine | Ile | I | $-CH(CH_3)CH_2CH_3$ |
| Leucine | Leu | L | $-CH_2CH(CH_3)_2$ |
| Lysine | Lys | K | $-(CH_2)_4NH_3^+$ |
| Methionine | Met | M | $-(CH_2)_2SCH_3$ |
| Phenylalanine | Phe | F | $-CH_2$-Phenyl |
| Proline | Pro | P | $-\alpha$,N-$(CH_2)_3$- |
| Serine | Ser | S | $-CH_2OH$ |
| Threonine | Thr | T | $-CH(OH)CH_3$ |
| Tryptophan | Trp | W | $-CH_2$-indole |
| Tyrosine | Tyr | Y | $-CH_2$-phenyl-OH |
| Valine | Val | V | $-CH(CH_3)_2$ |

| | |
|---|---|
| antibody | a protein produced by mamalian B cells that is capable of binding a particular antigen |
| ARC | AIDS-Related Complex |
| AZT | Azidothymidine, an anti-AIDS compound |
| bigeneric spacer | a molecular chain resulting from the reaction of separately derivatized partners; analytical degradation of the conjugate formed through the spacer allows release and quantitation of the spacer, providing a measure of the degree of covalent attachment |
| BOP | Benzotriazol-1-yloxytris(dimethylamino)phosphonium hexafluorophosphate |
| capping | the elimination of reactive sites on a conjugate by reaction with small molecules |
| Cbz | benzyloxycarbonyl |
| conjugate | a complex of discrete chemical entities covalently bound one to the other, wherein at least one entity is a desired antigen (e.g. an HIV PND) and another entity is a carrier |
| Core amino acids | those amino acids of an HIV PND which are essential for inducing HIV-neutralizing immune responses in a mammal |
| DPPA | diphenylphosphorylazide |
| ELF | epitopic loading factor; defined as the quantity, in milligrams, of a peptide epitope present per milligram of carrier protein, and may be calculated by obtaining the product (PF x GF), or more directly by quantitating a marker amino acid, such as norleucine or ornithine, and calculating the ratio of peptide to total protein mass in the conjugate |
| ELISA | enzyme-linked immunosorbant assay |
| Fmoc | 9-fluorenylmethyloxycarbonyl |
| GF | glycosylation factor; defined as the milligrams of polysaccharide per milligram of protein present in a conjugate |
| HIV | Human Immunodeficiency Virus, a member of the lentivirus group and the purported etiologic agent implicated in AIDS and related complexes; HIV is alternatively known as HTLV III (Human T-cell Lymphocyto-trophic Virus III), LAV (Lymphadenopathy Associated Virus), and ARV (AIDS Related Virus) |
| HIV disease | clinically recognized disease state characterized by presence of any of a number of physiologic dysfunctions known to be associated with HIV infection |
| immunogen | a molecule useful as a stimulator of a mammalian immune response |
| immunologically equivalent peptides | cyclic or linear peptides capable of eliciting HIV neutralizing immune responses in mammals, including antibodies which are able to bind HIV principle neutralizing epitopes |
| marker amino acid | an amino acid having a signal in the AA assay which is free of interference by signals generated by other peptide or protein amino acids; examples are norleucine (Nle), ornithine, β-alanine, gamma amino butyric acid (GABA) |
| Mtr | 4-methoxy-2,3,6-trimethylphenyl sulfonyl |
| NEM | N-ethylmaleimide |
| OMPC | Outer Membrane Protein Complex of Neisseria meningitidis; used as an immunoenhancer and peptide carrier |
| peptide | a polymer of amino acids linked by amide (peptide) bonds |
| PEP | peptide |
| PF | peptidylation factor; defined as the milligrams of peptide per milligram of polysaccharide present in a conjugate |
| PnPs | Streptococcus pneumonia copsulor polysacchoride; a number following this abbreviation, as in PnPs6B, indicates the strain from which the polysaccharide was derived.. |
| PND | Principal Neutralizing Determinant; the name attributed to peptidyl sequences capable of binding to HIV neutralizing antibodies and capable of raising HIV-neutralizing antibodies in a mammalian recipient upon inoculation with an immunogen containing the PND |
| PRO | an immunogenic protein |
| protein | a large peptide |
| PRP | Polyribosyl-ribitol phosphate |
| PSA | anionic polysaccharide, usually having repeat phosphate units in the monomer unit of the polymer |
| resins | solid support matrices for solid phase peptide synthesis<br>Wang:<br>4-(hydroxymethyl)phenoxymethyl linkage to copolystyrene-1%divinylbenzene resin, |

| | |
|---|---|
| | which is used for batch Fmoc solid phase peptide synthesis, with final 95% TFA cleavage from the resin and concomitant deprotection of acid sensitive side chain protecting groups; |
| | Sasrin: |
| | 4-(hydroxymethyl)-3-methoxyphenoxymethyl linkage to copolystyrene-1%divinylbenzene resin, which is used for batch Fmoc solid phase peptide synthesis, with final 1% $TFA/CH_2Cl_2$ cleavage from the resin, leaving intact acid labile side chain protecting groups; |
| | Pepsyn KA: |
| | 4-(hydroxymethyl)phenoxymethyl linkage to polyamide resin adsorbed on to kieselguhr, which is used for continuous flow column Fmoc solid phase peptide synthesis. Peptides are cleaved from the resin as described above for Wang resin; |
| | Pepsyn KH: |
| | 4-(hydroxymethyl)-3-methoxymethyl linkage to polyamide resin adsorbed on to kieselguhr, which is used for Fmoc solid phase peptide synthesis. Side chain protected peptides are cleaved from the resin as described above for the Sasrin resin |
| "scaffold" | immunogen having multiple peptide epitopes built upon a carrier molecule |
| SCMHC | S-carboxymethyl homocysteamine, an acid-stable bigeneric spacer released by degradation of covalent conjugate immunogens and quantifiable by AA assay |
| SCMC | S-carboxymethyl cysteamine, an acid-stable bigeneric spacer released by degradation of covalent conjugate immunogens and quantifiable by AA assay |
| Z | benzyloxycarbonyl |

## DETAILED DESCRIPTION OF THE INVENTION

### I. The Conjugation Process and the Conjugate Produced Thereby, and Utility of the conjugate:

The novel conjugates of the invention are prepared by the process of covalently coupling separately-activated peptide, polysaccharide, and protein components to each other. The peptide and protein components are similarly activated, to display either pendant electrophilic or nucleophilic groups, and they cannot react with each other. The polysaccharide component is conversely activated so as to display pendant nucleophilic or electrophilic groups, thus allowing reaction with the oppositely-activated peptides and proteins. Each reactive event results in the formation of a stable, covalent spacer, termed a bigeneric spacer because of the bimolecular process involved in its formation, that bonds either peptide or protein to the polysaccharide. The covalent conjugate immunogens that result from this series of reactions may conveniently be thought of as a scaffold in which multiple peptide moieties are built upon a foundation of polysaccharide which is in turn anchored to protein moieties.

When the peptide components are immunologically equivalent to PNDs of HIV, conjugates produced by this process may be administered to mammals in immunologically effective amounts, with or without additional immunomodulatory, antiviral, or antibacterial compounds, and can be used to induce mammalian production of anti-peptide antibodies, to induce mammalian production of HIV neutralizing antibodies, or to formualte vaccines to prevent human contraction of HIV infection or disease, including AIDS, or to treat humans afflicted with HIV infection or disease, including AIDS.

The level of activation of the polysaccharide may be titrated and an appropriately limited amount of peptide added to the conjugation reaction such that residual active sites remain on the polysaccharide even if all the added peptide becomes conjugated to the polysaccharide. These residual active sites on the polysaccharide may then be reacted with a protein activated in the same manner as was the peptide, to generate the peptide-polysaccharide-protein conjugates of this invention.

The final preparative step is elimination of residual electrophilic or nucleophilic sites present on the conjugate. This is accomplished by capping electrophilic sites with a low molecular weight nucleophilic reagent, preferably N-acetyl cysteamine (NAC). Residual nucleophilic sites, such as free sulfhydryl groups, are capped with a low molecular weight electrophile, preferably N-ethylmaleimide (NEM). Following this capping treatment, the conjugate may be isolated from reactants by differential centrifugation.

Chemical and physical characteristics of the covalent conjugate immunogens may be accurately and conveniently established. The mass ratio of peptide to polysaccharide and polysaccharide to protein may be determined at appropriate stages during the conjugation process. Total acid hydrolysis, either of the peptide-polysaccharide conjugate, or of the final peptide-polysaccharide-protein conjugate yields free amino acids and acid stable portions of the bigeneric spacers such as S-(carboxymethyl)cysteamine (SCMCA) or S-(carboxymethyl)homocysteine (SCMHC). An AA assay then allows quantitation of the unusual and isolated signal

elicited by the bigeneric spacers, thereby allowing direct quantitation of the number of covalent spacers present in the assayed conjugate aliquot. Since the amino acids comprising the peptide and protein may be quantitated in the same assay, a ratio of spacer to amino acid is easily calculated.

A monosaccharide assay, such as a ribose assay [for example Dische and Schwartz, Mikrochemica-acta 2, 13 (1937)], and a separate amino acid analysis (AA assay) of post peptide-polysaccharide conjugation aliquots yields a peptidylation factor (PF) defined as the milligrams of peptide per miligram of polysaccharide present. A subsequent total protein assay coupled with a monosaccharide assay of aliquots of the final conjugates yields a glycosylation factor (GF) defined as the milligrams of polysaccharide per milligram of protein present. By obtaining the product (PF x GF), the epitopic loading factor (ELF), defined as the milligrams of peptide per milligram of protein present, may be calculated. An alternate way of generating ELF is by inclusion in the peptide of a unique amino acid that is not present anywhere else in the peptide or protein moieties. Thus, inclusion of norleucine anywhere in a peptide, preferably away from the -GPG- trimer, and most preferably at the amino terminus of the peptide, is used herein as an facile way to measure total peptide in the conjugates of this invention.

The conjugate immunogen produced by the process outlined above may be further delineated by the structure IV and the critical parameters, PF, GF, and ELF further defined thereby:

$$\left[\left[PEP-r\right]_{PF}-PSA-B\right]_{GF}-PRO \qquad \underline{IV}$$

wherein:

PEP represents covalently coupled HIV PND peptide, having either a linear or a cyclic structure;
PSA represents anionic polysaccharide;
PRO represents an immunogenic protein or protein complex, and preferably the outer membrane protein complex (OMPC) of Neisseria meningitidis;
-r- represents a spacer forming a covalent linkage between the peptide and polysaccharide of the conjugate;
-B- represents a spacer forming a covalent linkage between the polysaccharide and protein of the conjugate;
PF = 0.001 to 10, and preferably about 0.01 to 1 milligrams PEP per milligram PSA;
GF = 0.001 to 10, and preferably about 0.01 to 0.33 milligrams PSA per milligram PRO;
ELF= epitopic loading factor, preferably in the range 0.0001 to 0.5 milligrams PEP per milligram PRO.

The parameters PF, GF, and ELF are manipulable simply by changing the degree of derivatization of the component species to allow optimization of the desired immune response.

The chemistry involved in the formation of spacers -r- and -B- in formula IV representing covalent conjugates of this invention may depend on non-specific cross-linking or on the use of "monogeneric" or "bigeneric" spacers [see Marburg et al., J. Am. Chem. Soc., 108, 5282 (1986)]. Preferably, the bigeneric spacer technology developed by Marburg is utilized. Once the conjugate has been prepared according to the bigeneric methodology, the conjugate may be hydrolyzed and analyzed. Acid-stable portions of the spacers -r- and -B- may then be quantitated as marker amino acid derivatives that show up in a window in the amino acid analysis spectrum (AA assay), free of interference by other amino acids. For example, according to the chemistry presented in SCHEME A below, acid hydrolysis of the conjugate releases the unique amino acid S-(carboxymethyl)cysteamine (SCMCA = $NH_2(CH_2)_2$-S-$CH_2COOH$) as the acid-stable portion of -r- and -B-. Likewise, according to SCHEME B, the unique amino acid S-(carboxymethyl)homocysteine (SCMHC = $HOOCCH_2S(CH_2)_2CHNH_2CO$-OH) is released from -r- and -B- upon acid hydrolysis of that conjugate. Naturally, other amino acids may be produced by altering the chemistry whereby the spacers -r- and -B- are formed, but the principle of formation and analysis are the same as discussed here.

In a preferred embodiment, conjugates of this invention may be prepared according to the steps outlined in Schemes A or B below. Scheme A depicts a process wherein the PSA is bromoacetylated while the PRO and PEP reaction partners are activated to exhibit pendant sulfhydryl groups. Conversely, Scheme B depicts a process wherein the PSA is activated to exhibit pendant sulfhydryls, and the PRO and PEP reaction partners are bromoacetylated. Other nucleophilic or electrophilic groups may be used to effect the conjugation chemistry [Marburg et al., J. Am. Chem. Soc. 108, 5282 (1986); U.S. Patent # 4,695,624].

A. Preparation of Peptide-PSA-PRO Conjugates (see Scheme A) - Thiolated PSA:

The preparation takes place in essentially three steps:

1. Anionic polysaccharide, PSA, may be isolated from cultures of gram- negative bacteria whose capsular polysaccharide is composed largely of an anionic polysaccharide polymer. For example, crude preparations of the polysaccharide isolated from fermentation culture supernatant of Haemophilus influenzae b or Streptococcus pneumoniae 6B, 19F, 23F, among others, may be lyophilized to afford powdered polyribosyl ribitol phosphate PRP or powdered PnPs6B, PnPs19F, or PnPs23F polysaccharide respectively. The dry powder that results is then solubilized in water, and acidic hydrogens are exchanged for hydrophobic ions, preferably cations such as tri- or tetrabutylammonium by passage of the PSA through a cation exchange column charged with tri- or tetrabutylammonium ions as in Marburg et al., J. Am. Chem. Soc., 108, 5282-5287 (1986), and U.S. Patent 4,695,624. The PSA that elutes may be lyophilised and resuspended in an organic solvent, preferably DMF or DMSO, and activated by a bis electrophile, such as a carbonic acid derivative, preferably carbonyldiimidazole ($COIm_2$). The resulting imidazoylurethane is subjected to nucleophilic attack, preferably by a thiol generating diamine, and most preferably by cystamine. The resulting titer of amine-derivatized PSA may be quantitated by a fluorescamine assay as in Udenfriend et al., Science 182, 871 (1972). According to Scheme A, X moles of the cystamine-functionalized PSA, IA, are reduced by treatment with dithiothreitol (DTT), dithioerythreitol (DTE), or a similarly strong reducing agent, and the small molecules, such as cleaved 2-aminoethane-thiol, are removed by dialysis or diafiltration under an atmoshphere of nitrogen. The resultant solution of X moles of IIA is assayed as in Ellman, G. L., Arch Biochem. Biophys. 82, 70 (1959), to determine the magnitude of X.

2. Y moles of a N-bromoacetylated peptide, or a mixture of different bromoacetylated PND peptides, are prepared by reaction of the peptide with a bromoacetyl donor, preferably p-nitrophenyl bromoacetate, followed by reisolation of the bromoacetylated peptide by reverse phase HPLC. The Y moles of bromoacetylated peptide is reacted with X moles of PSA-SH, IIA, such that Y is between about 10% and 80% of X, and preferably in an amount equivalent to about one half of the assayed thiol titer of IIA (i.e., Y = 0.5 X). After this reaction, the remaining thiol titer is determined by Ellman assay. By difference, the amount of peptide bound to the polysaccharide at this point may be inferred. The value thus obtained may be used to confirm the value obtained directly as described in (ii) below. The reaction product is characterized in two additional ways: (i) By conducting a monosaccharide assay of the PSA. Many monosaccharide assays are known in the art. For example, a ribose-sensitive method may be utilized to quantitate PRP and other ribose containing polysaccharides [Dische and Schwartz, Mikrochemica-acta 2, 13 (1937)]; and (ii) by acid hydrolysis followed by AA assay affording, via the quantitative S-carboxymethyl cysteamine (SCMC) value thus obtained, a titer of covalent peptide. As mentioned above, this value may be confirmed by the difference between the thiol titer by Ellman assay between IIA and IIIA. Alternatively, where an amino acid such as norleucine has been incorporated into the peptide, direct AA assay quantitation of Nle may be used to calculate the mass of peptide in a conjugate, either to yield the PF or as discussed infra, to generate the more significant parameter, ELF, directly. Combining the results of these assays, a peptidylation factor (PF) may be derived with units as follows: PF = milligrams peptide/mg of PSA.

3. The resultant partially-thiolated, partially-peptidylated PSA, IIIA, is then reacted with n moles of a bromoacetylated PRO to afford the conjugate IVA, having m moles of PRO covalently bound to the PSA. Where m<(X-F), there will remain (X-Y-m) = z moles of reactive groups available on PSA that are neutralized by capping with NEM. After removal of insoluble material by low-speed centrifugation, unconjugated PSA is separated from the conjugate by ultracentrifugal pelleting of the macromolecular conjugate, leaving the unconjugated PSA in the supernatant. IVA may be recovered from the pellet by resuspension in aqueous media, sterile filtered, and then may be assayed by a PSA and a Lowry protein assay to generate the glycosylation factor, GF = mg PSA/mg PRO. Using GF and the PF determined in section 2 above, ELF may be calculated as ELF = (GF x PF) = milligrams of peptide antigen per milligram of PRO.

# SCHEME A

## THIOLATED PSA

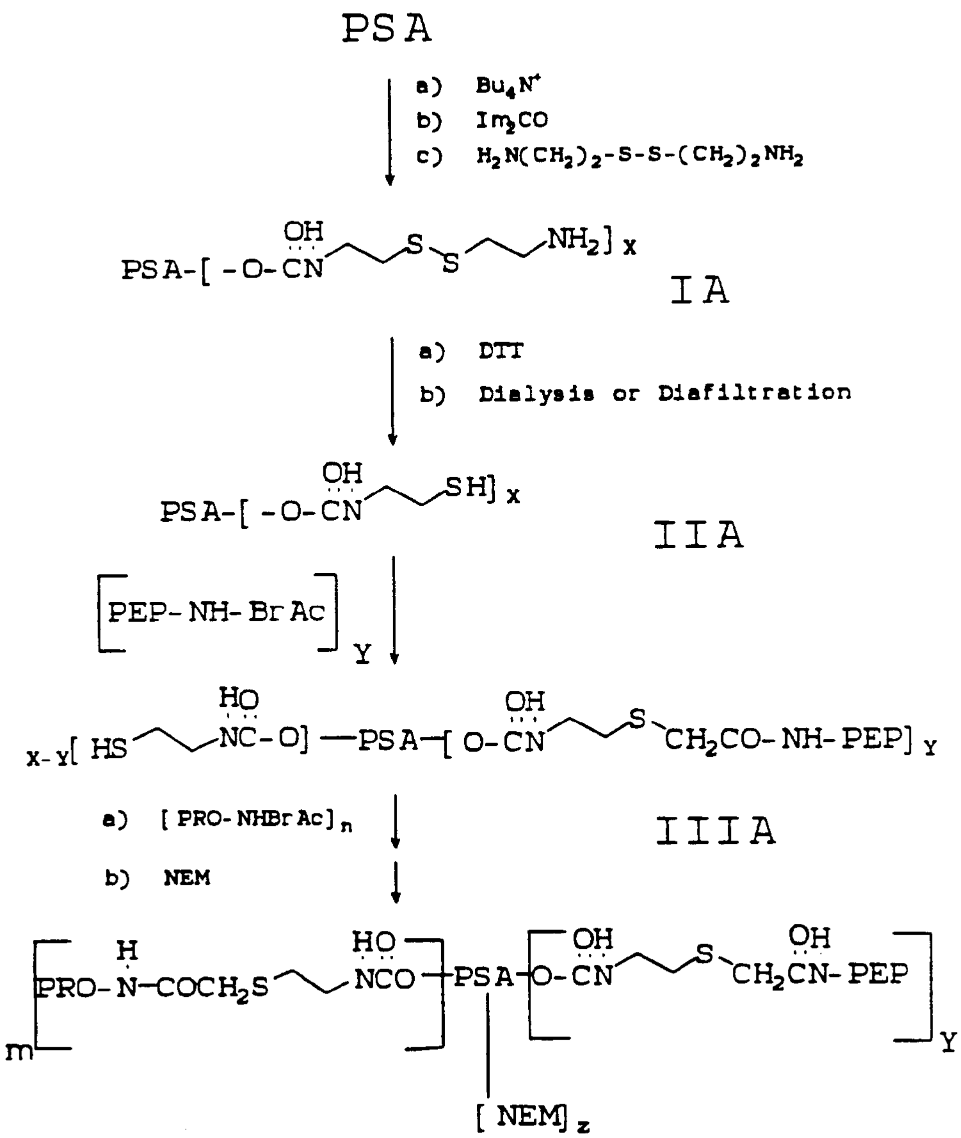


NOTE: m + z = (X - Y)

B. Preparation of Peptide-PSA-PRO Conjugates (see Scheme B) - Bromoacetylated PSA:

The preparation takes place in essentially three steps:

1. The first step is identical to the description in step A-I. above, up to the point of reaction with cystamine. Here, however, the PSA is reacted instead with a bis-nucleophile, preferably a diamine, and most preferably with butanediamine. The resulting titer of amine-derivatized PSA, IB, may be quantitated by a fluorescamine assay [Udenfriend et al., Science 182, 871 (1972)]. To X moles of IB, excess bromoacetyl donor, preferably p-nitrophenyl bromoacetate is added, to generate IIB having X moles of available reactive bromoacetyl groups.

2. Thiolated peptide, PEP-SH, may be prepared as the Ac-Cys(Acm) peptide having the thiol protected amino acid as the peptide amino-terminus. Upon cleavage of the Acm protecting group from the Ac-Cys(Acm) side chain [Atherton, E., et al., Chem. Soc. Perkin Trans., I, 2057 (1985)], the free sulfhydryl is revealed for reaction with bromoacetylated-PSA. Y moles of PEP-SH, wherein Y is between about 10% and 80% of X, and preferably about 50% X, including a mixture of different PEP-SH species, are reacted with X moles of bromoacetylated PSA, IIB, to generate the intermediate IIIB. Thus, IIIB has Y moles of conjugated PEP and (X-Y) moles of residual reactive bromoacetyl groups available for reaction with the PRO-SH.

3. The resultant partially-bromoacetylated partially-peptidylated PSA, IIIB, is then reacted with n moles of PRO-SH, affording the conjugate IVB containing m moles of conjugated PRO. Where m<(X-Y), because, for example, steric hinderance renders some bromoacetylated sites unavailable for reaction with PRO-SH, a capping step is necessary to remove these active sites. A small nucleophilic reagent, preferably N-acetylcysteamine (NAC), is used to neutralize these residual active sites. As described in (A) above, the insoluble material is pelleted by low-speed centrifugation, following which the macromolecular conjugate is separated from unconjugated PSA by ultracentrifugation. IVB is recovered from the ultracentrifugation pellet by homogenization in an aqueous buffer following which the conjugate may be sterile filtered and assayed as described in (A), and the parameters GF and ELF determined.

## SCHEME B

### BROMOACETYLATED PSA

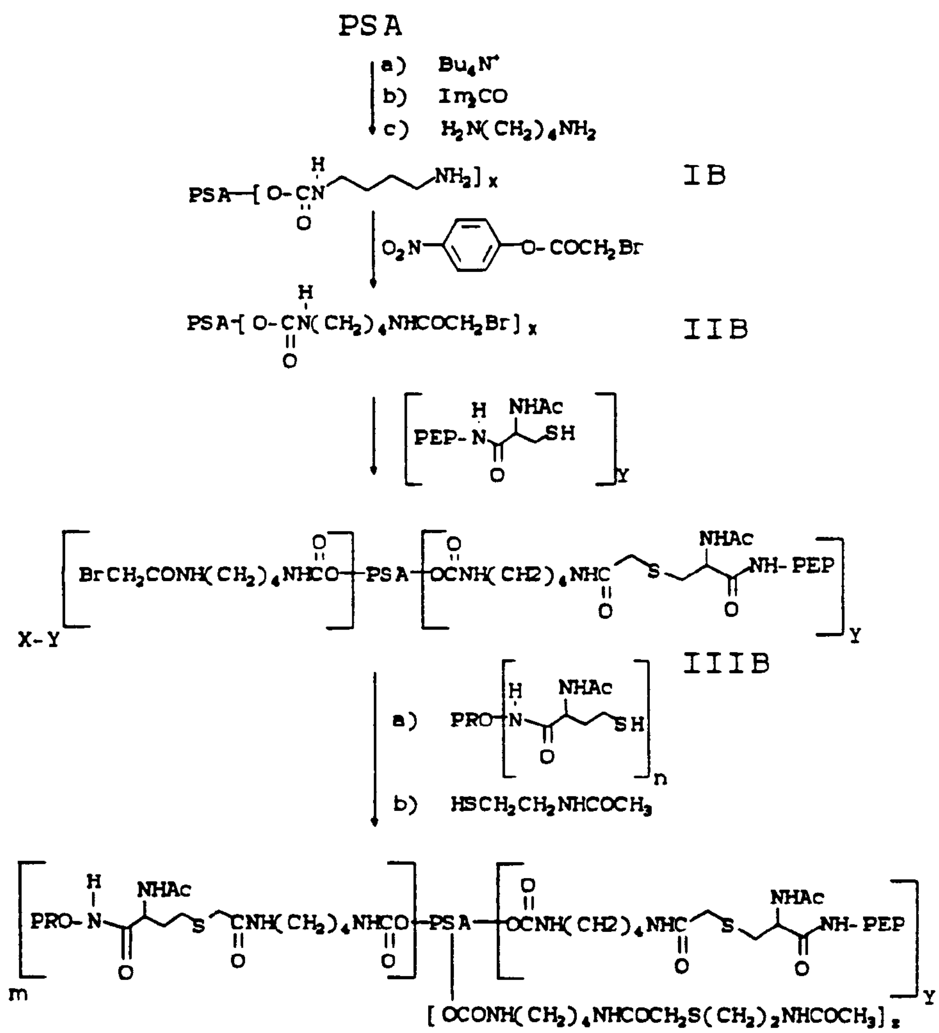


NOTE: m + z = (X - Y)

Thus, according to the methods of making the conjugates of this invention described supra, spacers -r- and -B- are formed which bind peptide to polysaccharide, and polysaccharide to protein respectively. Oxygen atoms from the PSA hydroxyls form urethane linkages to the spacer, while the free amino groups of the PEP or PRO moieties form amide bonds with the spacer. In a preferred embodiment or this invention, the spacers -r- and -B- are selected from:

a) $-CONH(CH_2)_4NHCOCH_2SCH_2CH(NHCOCH_3)CO-$,

b) $-CONH(CH_2)_4NHCOCH_2SCH_2CH_2CH(NHCOCH_3)CO-$, and

c) $-CONH(CH_2)_2SCH_2CO-$. The carbonyls of these spacers are either bonded to amines on peptides or proteins, to form amide bonds, or to oxygens on the polysaccharide to form urethane linkages. By modifying the conjugation chemistry (as per U.S. Patent 4,695,624), bigeneric spacers having substantially different structures may be prepared.

Upon acid hydrolysis, all acid labile bonds such as peptide (amide) bonds break and acid-stable portions of the spacer are released along with the peptide and protein constituent amino acids.

C. Utility:

The conjugates herein described may be included in compositions containing an inert carrier and are useful when appropriately formulated as a vaccine. This may include prior adsorption onto alum or combination with emulsifiers or adjuvants known in the art of vaccine formulation. Methods of using the covalent conjugate immunogens of this invention include: (a) use as a laboratory tool to characterize DIV PND peptide structure-function relationships; (b) use as an immunogen to raise HIV-neutralizing antibodies in a mammal which antibodies may be isolated and administered to a human so as to prevent infection by HIV, or to limit HIV proliferation post-infection, or to treat humans afflicted by HIV infection or disease, including AIDS; (c) use as a vaccine to immunize humans against infection by HIV or to treat humans post-infection, or to boost an HIV-neutralizing immune response in a human afflicted with HIV infection or disease, including AIDS.

As a laboratory tool, the conjugate is useful when administered to a mammal in an immunologically effective amount to generate anti-PND peptide, anti-HIV, or HIV-neutralizing immune responses. The mammal may be boosted with additional conjugate to elevate the immune response. Antiserum is obtained from such a mammal by bleeding the mammal, centrifuging the blood to separate the cellular component from the serum, and isolating antibody proteins from the serum if necessary, according to methods known in the art. Such antiserum or antibody preparations may be used to characterize the efficacy of an HIV PND peptide in a conjugate in raising mammalian anti-PND peptide, anti-HIV, or HIV-neutralizing antibodies in a mammal. ELISA assays using the unconjugated peptide and the antiserum are useful in vitro assays for measuring the elicitation of anti-peptide antibodies. An in vitro assay for measuring the anti-HIV, and HIV-neutralizing ability of antiserum comprises incubating a preparation of live HIV with a preparation of the antiserum, then incubating the antiserum-treated HIV preparation with CD4 receptor bearing cells, and measuring the extent of cellular protection afforded by the antiserum. These assays and the characteristics of antiserum produced by a given conjugate may be used to study the PND peptide stucture-function relationship.

The conjugate is useful for inducing mammalian antibody responses as described in the previous paragraph, and such antibodies may be used to passively immunize humans to prevent HIV infection, or to limit HIV proliferation post-infection, or to treat humans afflicted with HIV infection or diesease, including AIDS.

The conjugate is useful as a vaccine which may be administered to humans to prevent HIV infection or proliferation, or to humans suffering from AIDS and related complexes or humans testing seropositive for the HIV virus. The conjugate may be administered in conjunction with other anti-HIV compounds, such as AZT, or more general anti-viral compounds, or in conjunction with other vaccines, antibiotics, immunomodulators (see Table I below).

The form of the immunogen within the vaccine takes various molecular configurations. A single molecular species of the antigenic conjugate IV will often suffice as a useful and suitable antigen for the prevention or treatment of HIV disease, including AIDS or ARC. Other antigens in the form of cocktails are also advantageous, and consist of a mixture of conjugates that differ by, for example, the mass ratio of peptide to polysaccharide (PF), the mass ratio of polysaccharide to protein (GF), or the mass ratio of peptide to total protein (ELF). In addition, the conjugates in a mixture may differ in the amino acid sequence of the PND.

An immunological vector, carrier or adjuvant may be added as an immunological vehicle according to conventional immunological testing or practice.

Adjuvants may or may not be added during the preparation of the vaccines of this invention. Alum is the typical and preferred adjuvant in human vaccines, especially in the form of a thixotropic, viscous, and homogeneous aluminum hydroxide gel. For example, one embodiment of the present invention is the prophylactic vaccination of patients with a suspension of alum adjuvant as vehicle and a cocktail of conjugates as the selected set of immunogens or antigens.

The vaccines of this invention may be effectively administered, whether at periods of pre-exposure and/or post-exposure, in combination with effective amounts of the AIDS antivirals, immunomodulators, antibiotics, or vaccines of Table I [source: Market Letter, Nov. 30, 1987, p. 26-27; Genetic Engineering News, Jan. 1988, Vol. 8, p. 23.]

## TABLE I[1]

### A. Antivirals

| Drug Name | Manufacturer | Indication |
| --- | --- | --- |
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| FOSCARNET (trisodium phosphonoformate) | Astra AB | HIV inf, CMV retinitis |

---

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
| --- | --- | --- |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophosphoprotein) | Advanced Viral Research | AIDS, ARC |
| IR (zidovudine; AZT) | Burroughs Wellcome | AIDS, advanced ARC<br>pediatric AIDS, KS, asympt HIV, less severe HIV, neurological involvement. |
| RIFABUTIN (ansamycin LM 427) | Adria Labs | ARC |
| (trimetrexate) | Warner-Lambert | PCP |
| UA001 | Ueno Fine Chem Industry | AIDS, ARC |

| | | |
|---|---|---|
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| Drug Name | Manufacturer | Indication |
|---|---|---|
| ABPP (bropirimine) | Upjohn | Advanced AIDS, KS |
| AMPLIGEN (mismatched RNA) | DuPont HEM Research | ARC, PGL |
| (Anti-human alpha interferon antibody) | Advanced Biotherapy Concepts | AIDS, ARC, KS |
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |

| | | |
|---|---|---|
| IMREG-2 | Imreg | AIDS, ARC, PGL, KS |
| IMUTHIOL (diethyl dithio carbamate) | Merieux Institute | AIDS, ARC |
| IL-2 (interleukin-2) | Cetus | AIDS, KS |
| <u>Drug Name</u> | <u>Manufacturer</u> | <u>Indication</u> |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |
| MTP-PE (muramyl-tripeptide) | Ciba-Geigy | KS |
| THYMOPENTIN (TP-5) (thymic compound) | Ortho Pharmaceuticals | HIV infection |

| | | |
|---|---|---|
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |

C. Antibiotics

| | | |
|---|---|---|
| PENTAM 300 (pentamidine isethionate) | LyphoMed | PCP |

D. Vaccines

| | | |
|---|---|---|
| Gag | Merck | AIDS,ARC |

It will be understood that the scope of combinations of the vaccines of this invention with AIDS antivirals, immunomodulators, antibiotics or vaccines is not limited to the list in the above Table, but includes in principle any combination with any pharmaceutical composition useful for the treatment of AIDS. The AIDS or HIV vaccines of this invention include vaccines to be used pre- or post-exposure to prevent or treat HIV infection or disease, and are capable of producing an immune response specific for the immunogen.

The conjugates of this invention when used as a vaccine, are to be administered in immunologically effective amounts. Dosages of between 1 μg and 500 μg of conjugate protein, and preferably between 50 μg and 300 μg of conjugate protein are to be administered to a mammal to induce anti-peptide, anti-HIV, or HIV-neutralizing immune responses. About two weeks after the initial administration, a booster dose may be administered, and then again whenever serum antibody titers diminish. The conjugate should be given intramuscularly at a concentration of between 10 μg/ml and 1 mg/ml, and preferably between 50 and 500 μg/ml, in a volume sufficient to make up the total required for immunological efficacy. The conjugate may be preadsorbed to aluminum hydroxide gel or to the Ribi adjuvant (GB 2220211A, US priority document 212,919 filed 29/06/1988) and suspended in a sterile physiological saline solution prior to injection.

Preferred embodiments of the conjugate immunogen are further described hereinbelow with reference to the selection and preparation of each of the components required for making the conjugate immunogens of the invention.

II. Preferred Embodiments:

A. Selection and Preparation of the Polysaccharide, PSA:

The choice of polysaccharide carrier is a significant, as it is desirable that the immune response be primarily to the peptidyl determinants bound to the polysaccharide. In addition, it is necessary that no adverse reaction occur in the recipient in response to the polysaccharide portion of the conjugate. Some advantage may be gained by choosing PSA moieties which in themselves provide some protective benefit. For example, AIDS patients are at high risk of contracting infections induced by any of the subtypes of Streptococcus pneumoniae, commonly referred to as pneumococci (Pn), such as subtypes Pn6A, Pn6B, Pn10A, Pn11A, Pn18C, Pn19A, Pn20, Pn22F, and Pn23F. Thus, the use of capsular polysaccharide (PnPs) such as the PnPs6B, PnPS23F or the capsular polysaccharide from other such pathogenic organisms may, in addition to inducing immune responses against HIV, provide protection against deadly secondary bacterial infections. Some masking of the polysaccharide antigen can be expected, however, due to the appended peptide built upon the polysaccharide. Polyribosyl-ribitol phosphate (PRP) derived from Haemophilus influenzae b capsular polysaccharide, and certain of the pneumococcal capsular polysaccharides, including that derived from Pn6B, have proven suitable in preparing the conjugates of this invention.

A further consideration relevant to the choice of the polysaccharide is the need to neutralize excess charge that may be appended to the conjugate by the peptidyl moiety. In the case of the HIV peptides, a high incidence of arginine residues results in the addition of several positive charges per conjugated peptide. Therefore, a contribution made by the polysaccharide may be to neutralize the cationic peptides. Once again, PRP, with its motif of repeating anionic phosphate groups, and certain of the pneumococcal capsular polysaccharides, such as PnPs6B have proven suitable.

The PRP and pneumococcal polysaccharides may be derived by culturing Haemophilus influenza type b or certain subtypes of Streptococcus pneumoniae and the capsular polysaccharides isolated from the killed fermentation culture medium according to methods known in the art, and then prepared for conjugation as provided hereinabove and in the examples given hereinbelow.

B. Selection and Preparation of Immunogenic Protein, PRO:

The protein moiety should behave as an immune enhancer. It is desirable, in the choice of protein, to avoid those that result in non-specific activation of the recipient's immune response (reactogenicity). In U.S. Patent 4,695,624, Marburg et al. used the outer membrane protein complex (OMPC) derived from Neisseria meningitidis to prepare polysaccharide-protein conjugates. OMPC has proven suitable in this invention, though other immunogenic proteins may be used.

Various methods of purifying OMPC from the gram-negative bacteria have been devised [Frasch et al., J. Exp. Med. 140, 87 (1974); Frasch et al., J. Exp. Med. 147, 629 (1978); Zollinger et al., US Patent 4,707,543 (1987); Helting et al., Acta Path. Microbiol. Scand. Sect. C. 89, 69 (1981); Helting et al., US Patent 4,271,147]. OMPC used herein was prepared essentially according to the Helting process.

In addition, subunits of OMPC, such as the class 2 protein of Neisseria meningitidis, which is the major outer membrane protein [Murakami, K., et al., Infection and Immunity, 57, 2318 (1989)] , provide immune enhancement necessary to induce mammalian immune responses to HIV PND peptides. These subunits may be derived by dissociation of the isolated OMPC, or alternatively, produced through recombinant expression of the desired immunogenic portions of OMPC.

C. PND Peptides:

1. Definition of PND peptides:

PND peptides, both those known in the art and novel compounds herein disclosed are defined as peptidyl sequences capable of inducing an HIV-neutralizing immune response in a mammal, including the production of HIV-neutralizing antibodies.

A major problem overcome by the instant invention is the HIV interisolate sequence variability. The PND identified above occurs in the third hypervariable region of gp120, and although certain amino acids have been found to occur at given locations in a great many isolates, no strictly preserved primary sequence motif exists. This difficulty is surmounted by this invention because it allows conjugation of a cocktail of peptides having PND sequences from as many different HIV isolates as necessary to attain broad protection. Alternatively, a broadly- protective cocktail of conjugates may be prepared by mixing conjugates, each of which is prepared

separately with a peptide moiety providing protection against a single or several HIV isolates.

The amino acids found near or between amino acids 296 and 341 of gp120 have been shown to meet the criteria which define a PND. In the IIIB isolate of HIV, a 41-amino-acid sequence has been reported as follows (see ABBREVIATIONS AND DEFINITIONS for 3 letter codes and amino acid names): -INCTRPNNNTRKSIRIQRGPGRAFVTIGKIGNMRQAHCNIS-, with the two cysteines being disulfide bonded to each other to form a loop. The trimer -GPG- is exposed at the PND loop tip. Peptides from different HIV isolates from this same region of gp120 raise isolate-specific neutralizing antibodies when presented to goat and guinea pig immune systems as conjugates with keyhole-limpet hemocyanin. The major neutralizing epitope within the 41-mer sequence, presented above, is comprised by the eight amino acids surrounding and including the -GPG- trimer [Javaherian et al., PNAS USA 86, 6768 (1989)]. In Table II below a number of linear peptides of different length and composition that can be used to prepare the conjugates of this invention are presented. The name of the isolate containing a peptide having the sequence of the tabulated peptide is given, along with a name herein ascribed to that peptide for ease of reference. The letter r- on the left hand side of each peptide represents the possibility of linking the peptide to the PSA at that position. In addition, marker amino acids, such as norleucine and ornithine may form part of r-.

TABLE II

LINEAR HIV PND PEPTIDES

| HIV Isolate | Peptide Sequence | Name |
|---|---|---|
| MN | r-YNKRKRIHIGPGRAFYTTKNIIGT | PND142 |
| SC | r-NNTTRSIHIGPGRAFYATGDIIGDI | PND-SC |
| IIIB | r-NNTRKSIRIQRGPGRAFVTIGKIGN | PND135 |
| IIIB | r-RIQRGPGRAFVTIGKIGN | PND135-18 |
| IIIB | r-RIQRGPGRAFVT | PND135-12 |
| MN | r-HIGPGRAF | PND-MN8 |
| MN | r-GPGRAF | PND-MN6 |
| LAV-1 | r-IQRGPGRAF | PND-LAV-1 |
| SF2 | r-IYIGPGRAF | PND-SF2 |
| NY5 | r-IAIGPGRTL | PND-NY5 |
| CDC4 | r-VTLGPGRVW | PND-CDC4 |
| RF | r-ITKGPGRVI | PND-RF |
| ELI | r-TPIGLGQSL | PND-ELI |

| | | |
|---|---|---|
| Z6 | r-TPIGLGQAL | PND-Z6 |
| MAL | r-IHFGPGQAL | PND-MAL |
| Z3 | r-IRIGPGKVF | PND-Z3 |

This list is not an exhaustive list of possible PND sequences. Rather, it is provided as suggestive and illustrative guide as to useful PND primary sequences. Therefore, peptides conjugated as herein described to form the immunogen of this invention are any of the tabulated peptides or immunologically-equivalent variants on the theme suggested by these peptidyl sequences. The nature of the variations is considered next.

The primary sequence of this HIV PND appears to have a conserved core amino acid sequence, comprised by the tetramer sequence -GlyProGlyArg- (-GPGR-), with increacing divergence on either side of this sequence among HIV isolates. Some isolates have sequences that diverge even within the tetramer, having -GPGK-, -GPGQ-, and even -GLGQ- core sequences. All of these possible sequences come within the scope of this disclosure as being peptide sequences that are advantageous for conjugation according to this invention.

The length of the peptide is a significant factor in promoting cross reactive immune responses. That is, an immune response raised against a given peptidyl epitope may recognize similar epitopes from the same or different HIV isolate based on the number of amino acids in the epitope over and above the critical neutralizing epitope. In addition, the length of the peptide is also responsible for determining the probability of exposure to the immune system of the determinant responsible for generating an HIV-neutralizing response.

In order to maximize the probability of relevant epitope presentation, chemistry was developed whereby the PND peptides may be locked into a given three-dimensional configuration. It is known that the 41-amino-acid PND of the HIV IIIB isolate, represented above, is configured as a loop by the presence of the cysteine-to-cysteine disulfide bond. Disulfides, however, are labile and therefore may allow the loop to open and the peptide to exist in a linear form. Therefore, in addition to linear peptides, novel disulfide-bonded cyclic peptides and novel HIV PND peptides having nonlabile cyclic structures disclosed herein may all be utilized as the PEP component in the formation of the conjugates of this invention.

The peptides that may be used in formation of these conjugates may be derivedas fragments of natural proteins (gp120 for example), by recombinant expression of portions thereof, or by chemical synthesis according to Known methods in the art. In addition, novel cyclic PNDs may be prepared synthetically according to the processes herein described. The sequences may contain both natural L-amino acids, or unusual or D-amino acids. In addition, the conjugation chemistry is sufficiently flexible so that the appropriate choice of peptide derivatization reagents allows for successful conjugation.

Synthetic peptides have been prepared by a number of strategies conducted either in solution or on solid supports. Excellent texts covering the basic principles and techniques are: Principles of Peptide Synthesis, Bodanszky. M., Springer-Verlag (1984); Solid phase Peptide Synthesis, Stewart J. M., Young, J. D., Pierce Chemical Company (2nd. ed. 1984); and The Peptides, Gross, E., Meienhofer, J., Academic Press, Inc., (1979). The processes described herein, however, are not limited to the disclosure of these texts.

Synthetic cyclic peptides may be prepared in two phases. First, the linear peptide may be synthesized on a Milligen 9050 peptide synthesizer or an ABI 431A peptide synthesizer using 9-fluorenylmethyloxy-carbonyl (Fmoc) chemistry and side-chain-protected Fmoc-amino acid pentafluorophenyl esters which are known reagents, or using derivatized Wang resin, Fmoc chemistry, and side-chain protected Fmoc-amino acid symmetrical anhydrides, prepared in situ, as reagents.

Second, the linear peptide may be cyclized, either in solution or with the peptide still attached to the solid phase resin. Cyclization may be accomplished by any technique known in the art, which may comprise, for example: a) incorporating cysteine residues into the linear peptide on either end of the sequence which is to form the loop and allowing disulfide bond formation under oxidizing conditions known in the art; b) preparing a cysteine containing peptide as in (a) but retaining the cysteines as free sulfhydryls (or as Acm protected thiols which are deprotected to the free sulfhydryls) and treating the peptide with o-xylylene dibromide or similar reagent, such as the diiodide, dichloride, or a dihalogenated straight or branched chain lower alkyl having between two and eight carbon atoms; such reagents react with the sulfur atoms of the cysteines to form a cyclic structure containing two nonlabile thioether bonds to the benzene or the alkyl; c) allowing a free group on one side of the loop amino acids to become amide bonded to a free carboxyl group on the other side of the loop amino acids through DPPA, BOP, or similar reagent mediated peptide bond formation. Each of these strategies is

taken up in more detail below, after presentation of a generalized description of the cyclic peptides produced by these methods.

Thus, without limiting the conjugate invention to the following peptides or methods of producing them, the PND peptides which may be conjugated after removal of appropriate protecting groups as necessary, according to this invention include those represented by the structure PEP, which includes the linear peptides of Table II above:

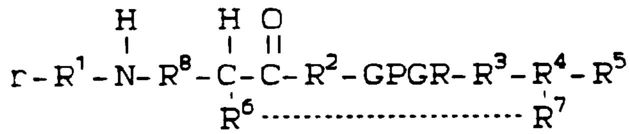

wherein:

r is the position of linkage between PEP and PSA, optionally comprising a marker amino acid, if $R^1$ is not a marker amino acid;

$R^1$ is:

a) a bond, or

b) a peptide of 1 to 5 amino acids, optionally including a marker amino acid which migrates at a position in the amino acid analysis spectrum which is isolated from the signal of the 20 naturally occuring amino acids; preferably norleucine, gamma aminobutyric acid, β-alanine, or ornithine;

$R^2$ is:

a) either a bond or a peptide of up to 17 amino acids if $R^3$ is a peptide of at least 2 amino acids, or

b) a peptide of between 2 to 17 amino acids, if $R^3$ is a bond;

$R^3$ is:

a) either a bond or a peptide of up to 17 amino acids if $R^2$ is a peptide of at least 2 amino acids, or

b) a peptide of between 2 to 17 amino acids, if $R^2$ is a bond;

-GPGR- is the tetramer -GlyProGlyArg-;

$R^4$ is:

a) -NH-CH-CO-, with $R^7$ bonded to the methine carbon, if $R^7$ is $R^8$, or

b) a bond from $R^3$ to $R^7$ and $R^5$,

if $R^7$ is 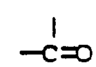 , or 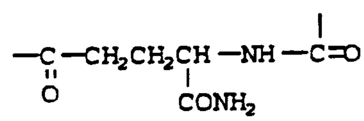 ;

$R^5$ is:

a) a peptide of one to five amino acids, optionally including a marker amino acid,

b) -OH,

c) -COOH,

d) $-CONH_2$,

e) $-NH_2$, or

f) -absent;

$R^6$ is:

a) an amino acid side chain, selected from the side chain of any of the common L or D amino acids, (see table of Definitions and Abbreviations), if the optional bond (--------) to $R^7$ is absent,

b) $-R^8$-S-S-, or $-R^8$-S-$R^8$-$R^9$-$R^8$-S-, if $R^7$ is $R^8$, or

c) $-R^8$-NH- if $R^7$ is

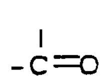 , or 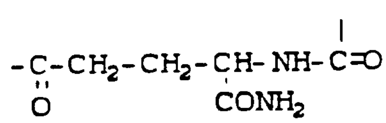 ;

$R^7$ is:

a) $-R^8-$,

b)

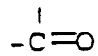 , or

c)

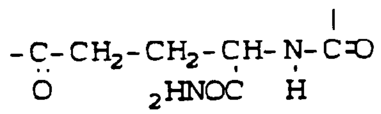 ;

$R^8$ is a bond or a lower alkyl of between one and eight carbons;

$R^9$ is:

a) $R^{10}$, or

b) xylylene;

$R^{10}$ is:

a) lower alkyl of between one and eight carbons, or

b) $-CH_2-O-CH_2-$; and every occurrence of a variable is independent of every other occurrence of the same variable. When a peptide has been synthesized with a protected amino terminal amino acid, the an amino terminal protecting group such as benzyloxy carbonyl (Z) for protecting amines, or acetamidomethyl (Acm) for protecting sulfhydryls, may be removed according to methods known in the art and exemplified herein. The deprotected group thus revealed may be utilized in conjugate bond formation, through the linker r, to the anionic polysaccharide, PSA.

Hereinafter, amino acids $-R^2$-GPGR-$R^3-$, which form the "core" of the PND peptides, and go toward formation of the loop of a cyclic peptide, will be referred to as loop amino acids. When the optional bond between $R^6$ and $R^7$ is absent however, the structure, PEP, is linear, and encompasses all of the linear peptides of Table II.

Whether the peptide is linear or cyclic, the amino acid sequences comprising $R^2$ and $R^3$ of PEP may be any combination of amino acids, including sequences surrounding the core -GPGR- tetramer in any of the sequences of Table II. Thus, the core amino acids represented by $-R^2$-GPGR-$R^3-$ may be further defined as having the core amino acid structure:

$-X_nX_1X_2$-GPGR-$X_3X_4X_m-$

wherein:

-GPGR- is the tetramer -GlyProGlyArg-;

$X_1$ is a constituent of $R^2$ selected from:

a) serine,

b) proline,

c) arginine,

d) histidine,

e) glutamine, or

f) threonine;

$X_2$ is a constituent of $R^2$ selected from:

a) isoleucine,

b) arginine,

c) valine, or

d) methionine;

$X_n$ is is a constituent of $R^2$ and is either a bond or a peptide of up to 15 amino acids;

$X_3$ is a constituent of $R^3$ selected from:

a) alanine,

b) arginine, or

c) valine;

$X_4$ is a constituent of $R^3$ and is selected from:

a) phenylalanine,

b) isoleucine,
c) valine, or
d) leucine;

$X_m$ is a constituent of $R^3$ and is a bond or a peptide of up to 15 amino acids.

In a preferred embodiment of this invention, $X_2$ is isoleucine, such that the PND is that of the MN isolate of HIV. It is also a feature of the preferred embodiment that the peptide contain a total of about 12-30 amino acid residues within the loop.

The cyclic peptides may be labile disulfide bonded structures or a cycle formed through a nonlabile bond or structure. The term "nonlabile bond" means a covalent linkage, other than a labile disulfide bond. Examples of such nonlabile bonds are amide and thioether bonds. These covalent linkages may be through a bridge structure, such as xylylene, or through a lower alkyl, or to an amino acid peptide-bonded bridge. By altering the bridge structure and/or the number and combination of amino acids included in the peptide, the conformation of the loop structure of the cycle may be optimized, allowing for fine-tuning of the PND epitope presented to the immune system. For example, use of an o-xylylene bridge generates a "tighter" loop structure than when, for example, an eight carbon straight chain lower lakyl is used as the bridge. Thus, the conjugates of this invention are useful both as reagents to analyze the structure-function relationship of the PND epitope in raising anti-peptide, anti-HIV, HIV-neutralizing, and anti-AIDS immune responses in mammals, and as components for formulation of anti-AIDS vaccines.

Synthetic products obtained may be characterized by fast-atom-bombardment mass spectrometry [FAB-MS], reverse phase HPLC, amino acid analysis, or nuclear magnetic resonance spectroscopy (NMR).

2. Processes for making Cyclic PND peptides:

a. Cyclic Peptides through Disulfide-Bonded Cysteines:

Peptides containing cysteine residues on either side of the loop amino acids may be cyclized under oxidizing conditions to the disulfide-bonded cycles. Methods for achieving disulfide bonding are Known in the art. An example of disulfide bonding within this invention is given infra in Example 5, wherein cPND4 is produced. In that example, a process utilizing the Acm derivative of cysteine to generate disulfide bonded cPNDs is used, but other processes are equally applicable. For example, in Example 43, cPND33 is prepared by highly diluting the linear peptide, having 2 free sulfhydryls, in trifluoroacetic acid. The peptide is allowed to form disulfides over 1-50 hours at between about 10 and 40°C. The disulfide bonded peptides are prefered.

Thus, in a preferred embodiment of this invention, the peptide has the structure:

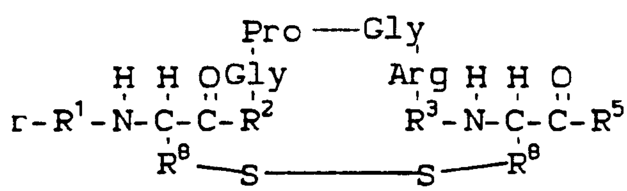

or pharmaceutically acceptable salts thereof, wherein:

r is:

a) hydrogen,

b)

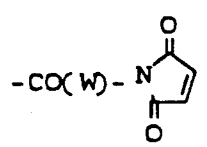 ,

wherein W is preferably $-(CH_2)_2-$ or $-(CH_2)_3-$ or $R^6$, where $R^6$ is

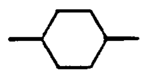 , 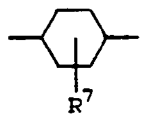 , 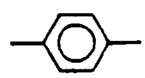 or 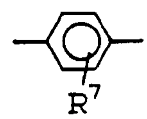

wherein $R^7$ is lower alkyl, lower alkoxy, or halo;

$R^1$ is:

a) a bond, or

b) a peptide of 1 to 5 amino acids, optionally including a marker amino acid;

$R^2$ is :

a peptide of 3 to 10 amino acids

$R^3$ is:

a peptide of 3 to 10 amino acids -GPGR- is the tetramer -GlyProGlyArg-;

$R^5$ is:

a) -OH,

b) a peptide of 1 to 5 amino acids, optionally including a marker amino acid, or

c) $-NH_2$;

$R^8$ is lower alkyl of between one and eight carbons.

Lower alkyl consists of straight or branched chain alkyls having from one to eight carbons unless otherwise specified. Hereinafter, amino acids $-R^2$-Gly Pro Gly Arg-$R^3$-, which go toward formation of the loop of a cyclic peptide, will be referred to as loop amino acids.

In one embodiment of the invention, the cyclic peptide having the structure:

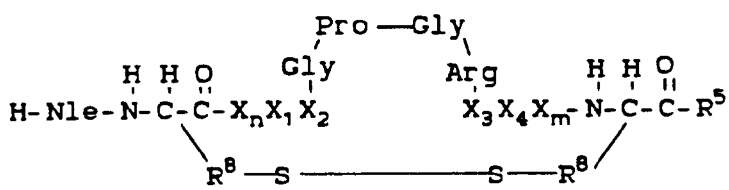

is prepared by cyclizing a linear peptide having the structure:

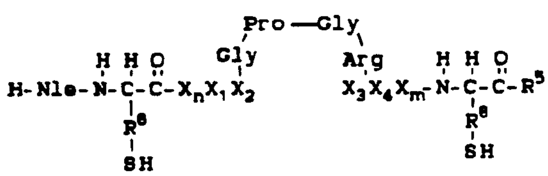

wherein:

$X_1$ is a constituent of $R^2$ selected from:

a) serine,

b) proline,

c) arginine,

d) histidine,

e) glutamine, which is preferred, or

f) threonine;

$X_2$ is a constituent of $R^2$ selected from:

a) isoleucine, which is most preferred,

b) arginine, which is preferred,

c) valine, or

d) methionine;

$X_n$ is a constituent of $R^2$ and is an amino acid or a peptide of up to 8 amino acids;

$X_3$ is a constituent of $R^3$ selected from:

a) alanine,

b) arginine, or

c) valine;

$X_4$ is a constituent of $R^3$ and is selected from:

a) phenylalanine,

b) isoleucine,

c) valine, or

d) leucine;

$X_m$ is a constituent of $R^3$ and is an amino acid or a peptide of up to 8 amino acids.

$X_2$ is preferably Isoleucine.

The novel disulfide bonded cyclic peptides used in this invention may be prepared in essentially two phases: First the linear peptide is synthesized on a Milligen 9050 or an ABI-431A peptide synthesizer using 9-fluorenyl-methyloxycarbonyl (Fmoc) chemistry and appropriately side-chain protected Fmoc-amino acid pentafluoro-phenyl esters as reagents or using derivatized Wang resin, Fmoc chemistry, and side-chain protected Fmoc-amino acid symmetrical anhydrides, prepared in situ, as reagents.

Second, the linear peptide is cyclized, either in solution or with the peptide still attached to the solid phase resin by incorporating cysteine residues into the linear peptide at either end of the sequence which is to form the loop, and oxidizing these to the disulfide. In a preferred embodiment, cyclization is accomplished by exposure of the peptide to (a) $H_2O_2$, (b) atmospheric oxygen, (c) aqueous $CH_3CN$ containing about 0.1 - 0.5% TFA, or (d) about 0.1M ferricyanide. The preferred method is exposure to atmospheric oxygen.

Products obtained may be characterized by fast atom bombardment-mass spectrometry [FAB-MS], reverse phase HPLC, amino acid analysis or nuclear magnetic resonance spectroscopy (NMR).

Thus, the peptides useful in this invention may be prepared as further described below in (i) and (ii):

i. Peptide Cyclization in the Solid State:

A linear peptide containing $C^1$ and $C^2$ on either side of the loop amino acids, where $C^1$ and $C^2$ are both cysteine or another amino acid containing free sulfhydryl groups in the side chain, is prepared according to known synthetic procedures (see discussion supra). In the completed cyclic PND, the sulfhydryl containing side chains, ($-R^8$-SH), go toward making up the $-R^8$-S- groups of the completed cyclic HIV PND structure shown above. Amino acids to be incorporated which have reactive side chains (R groups) are used in an appropriately R-group protected form. For example, histidine is triphenylmethyl (Trt), or Boc protected, and arginine is 4-methoxy-2,3,6-trimethylphenyl sulfonyl (Mtr) protected.

Preferably, a resin is purchased with $C^2$ in its Acm protected form already attached to the resin, for example, Fmoc-L-Cys(Acm)-O-Wang resin. The cysteine incorporated at the amino terminal side of the loop amino acids, $C^1$, may also be the Acm derivative. Either $C^1$ or $C^2$ may be bound to additional amino acids, $R^1$ or $R^5$ respectively, which may be utilized in the formation of conjugates with carrier molecules or may serve as marker amino acids for subsequent amino acid analysis, such as when norleucine or ornithine is used.

The sulfur of the acetamidomethylated cysteines are reacted, at room temperature for about 15 hours in a solvent compatible with the resin, as a 1-50% concentration of an organic acid, preferably about 10% acetic acid in anhydrous dimethylformamide (DMF), with about a four fold molar excess of a heavy metal salt, such as mercuric acetate [$Hg(OAc)_2$] for each Acm group. The resulting heavy metal thioether, for example the mercuric acetate thioether of the peptide, PEP(S-HgOAc), is then washed and dried. Addition of excess hydrogen sulfide in DMF yields insoluble metal sulfide, e.g. mercuric sulfide (HgS), and the peptide with free sulfhydryl groups. The free sulfhydryls are then oxidized by one of the aforementioned methods. Alternatively, the Acm protected thiols may be converted directly to the cyclic disulfide by treatment with iodine in a methanol/DMF solvent.

ii. Cyclization of Peptides in Solution:

Essentially the same process described above for solid state cyclization applies with two main variants: If the peptide is cleaved (95% TFA/4% ethanedithiol/1% thioanisole) from a pepsyn KA resin, acid labile side chain protecting groups are also removed, including Cys(Trt) which provides the necessary free -SH function. If however, Cys(Acm) protection is used, then mercuric acetate/hydrogen sulfide cleavage to the free -SH group is required as an independent procedure, with the linear peptide either on or off the resin.

One method however, is the use of Cys(Acm) protection and Sasrin or Pepsyn KH resin, and cleavage of the linear, fully protected peptide from the resin with 1% $TFA/CH_2Cl_2$. Mercuric acetate/hydrogen sulphide then selectively converts Cys(Acm) to the free -SH group, and cyclization is effected on the otherwise protected peptide. At this point, the peptide may be maleimidated in situ , selectively on the N-terminus. Acid labile side chain protecting groups are cleaved with 98% TFA/2% thioanisole, and the cyclic peptide is isolated by HPLC. The

preferred method, however, is to cleave the peptide from the resin, and allow cyclization by one of the aforementioned methods. The most preferred method is to allow air oxidation for about one to fifty hours of between 10 and 40°C.

Thus, in a particularly preferred embodiment of this invention, a peptide (cPND 33) having the structure (SEQ ID: 1:):

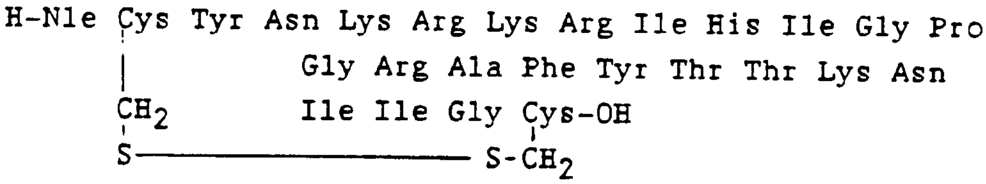

is conjugated to OMPC through either the amino terminal Nle or one of the internal lysines to generate one or a mixture of all of the structures:

a) 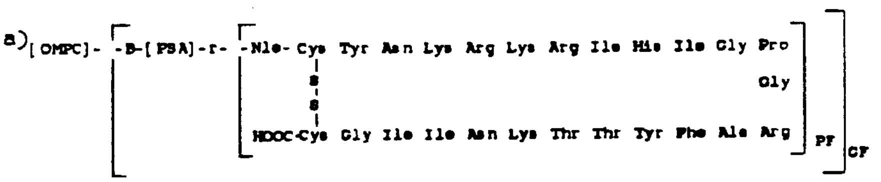 .

b) 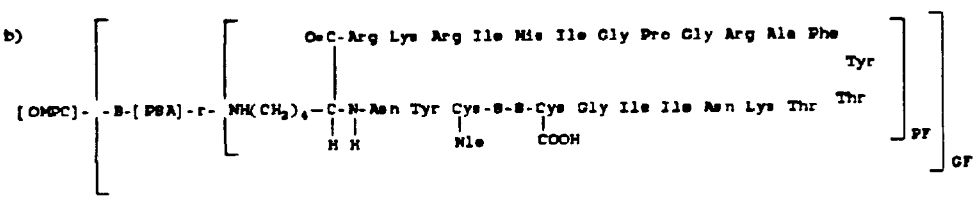 .

c) 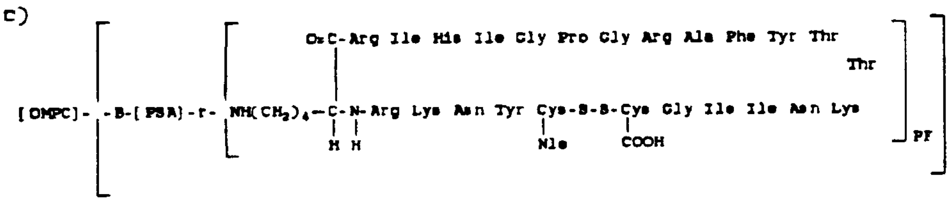 . or

d) 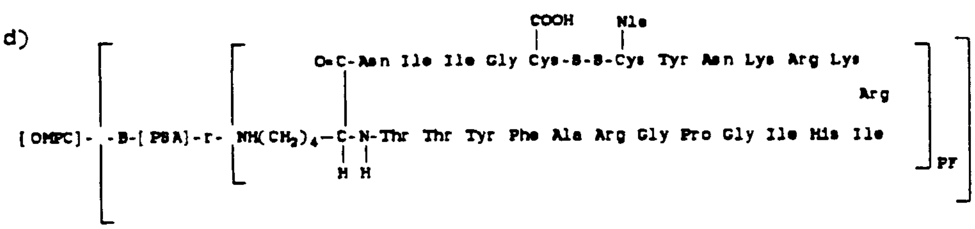 .

wherein:
-r- and -B- are selected from:
a) $-CONH(CH_2)_4NHCOCH_2SCH_2CH(NHCOCH_3)CO-$,
b) $-CONH(CH_2)_4NHCOCH_2SCH_2CH_2CH(NHCOCH_3)CO-$, or
c) $-CONH(CH_2)_2SCH_2CO-$;
PSA is comprised of either PRP or PnPs6B;
PF indicates a mass ratio of between 0.01 and 1 milligrams of peptide per milligram of PRP or PnPs6B; and
GF indicates a mass ratio of between 0.01 and 0.33 milligrams of PRP or PnPs6B per milligram of OMPC.

b. Cyclic Peptides through Thioether Linkage to o-Xylylene or Lower Alkyls:

i. Peptide Cyclization in the Solid State:

A linear peptide containing $C^1$ and $C^2$ on either side of the loop amino acids, where $C^1$ and $C^2$ are both cysteine or another sulfhydryl containing amino acid, is prepared according to known synthetic procedures (see discussion supra). In the completed cylic PND, $C^1$ and $C^2$ become part of the $R^6$ and $R^7$ groups of the PEP structure shown above. Amino acids to be incorporated which have reactive side chains (R groups) are used in an appropriately R-group-protected form. For example, histidine is triphenylmethyl- (Trt) protected, arginine is 4-methoxy-2,3,6-trimethylphenyl sulfonyl (Mtr) protected. [Principles of Peptide Synthesis, Bodanszky. M., Springer-Verlag (1984); Solid Phase Peptide Synthesis, Stewart J. M., Young, J. D., Pierce Chemical Company (2nd. ed. 1984); and The Peptides, Gross, E., Meienhofer, J., Academic Press, Inc., (1979)].

Preferably, a resin is purchased with $C^2$ in its Acm-protected form already attached to the resin, for example, Fmoc-L-Cys(Acm)-O-Wang resin. The cysteine incorporated at the amino terminal side of the loop amino acids, $C^1$, may also be the Acm derivative. Either $C^1$ or $C^2$ may be bound to additional amino acids, $R^1$ or $R^5$ respectively, which may be utilized in the formation of conjugates with carrier molecules or may serve as marker amino acids for subsequent amino acid analysis, such as when norleucine or ornithine is used.

The sulfur of the acetamidomethylated cysteines is reacted, at room temperature for about 15 hours in a solvent compatible with the resin, such as 10% acetic acid in anhydrous dimethylformamide (DMF), with about a four-fold molar excess of a heavy metal salt, such as mercuric acetate [$Hg(OAc)_2$] for each Acm group. The resulting heavy metal thioether, for example the mercuric acetate thioether of the peptide, PEP(S-HgOAc), is then washed and dried. Addition of excess hydrogen sulfide in DMF yields insoluble metal sulfide, e.g., mercuric sulfide (HgS), and the peptide with free sulfhydryl groups.

A mixture of about an equimolar amount, as compared with peptide, of o-xylylene dibromide or dichloride, or a dibrominated or dichlorinated lower alkyl, or similar reagent, such as dihalogenated $-CH_2-O-CH_2-$, which will provide a desirable bridge length, is added to the derivatized resin. A large excess of tertiary amine, preferably triethylamine ($NEt_3$) in DMF is added slowly. The reaction with the bis-sulfhydryl peptide moiety is allowed to proceed for about sixteen hours at room temperature, yielding the bridge group derivatized cyclic peptide bound to resin. Deprotection of acid sensitive side chain protecting groups and cleavage from the resin is achieved by treatment with 95% trifluoroacetic acid (TFA) in the presence of 4% 1,2-ethanedithiol and 1% thioanisole. The dissolved cyclic peptide may then be separated from the resin by filtration. The filtrate is evaporated and the crude residual product is purified by high performance liquid chromatography (HPLC) according to known methods, for example by reverse phase HPLC.

ii. Cyclization of Peptides in Solution:

Essentially the same process described above for solid state cyclization applies with two main variants: If the peptide is cleaved (95% TFA/4% ethanedithiol/1% thioanisole) from a pepsyn KA resin, acid labile side chain protecting groups are also removed, including Cys(Trt) which provides the necessary free -SH function. If however, Cys(Acm) protection is used, then mercuric acetate/hydrogen sulfide cleavage to the free -SH group is required as an independent procedure, with the linear peptide either on or off the resin.

The preferred method however, is the use of Cys(Acm) protection and Sasrin or Pepsyn KH resin, and cleavage of the linear, fully protected peptide from the resin with 1% $TFA/CH_2Cl_2$. Mercuric acetate/hydrogen sulphide then selectively converts Cys(Acm) to the free -SH group, and cyclization is effected on the otherwise protected peptide. Acid labile side chain protecting groups are cleaved with 95% TFA/4% ethanedithiol/1% thioanisole, and the cyclic peptide is isolated by HPLC.

Removal of excess reagents, such as unreacted xylylene dibromide, prior to acid cleavage of the protecting groups is conveniently achieved by, for example, a step gradient reverse phase HPLC run prior to more selective gradient elution.

Cyclic HIV PND peptides prepared according to the method of this subsection include, but are not limited to, the sample cPNDs represented below. The methods of this subsection are generally applicable to small peptides, and particularly applicable to peptides of between 5 and 30 amino acids. An optimal ring size may include between 5 and 10 amino acids, including the -GPG-trimer, and this ring size is easily maintained by production of cycles from linear peptides having the appropriate number and combination of amino acids.

Representative peptides resulting from the process described in this subsection b. parts (i). and (ii) are shown below. The conjugate invention should, however, not be construed as being limited to use these particular embodiments of HIV cyclic PND peptides. Other linear HIV PND peptide sequences may be cyclized in essentially the same fashion used to provide these peptides. Series of peptides having divergent primary sequences could be generated and would be beneficial in this invention as long as they continue to elicit an anti-peptide, anti-HIV, or HIV-neutralizing immune response.

| Name | Structure |
|---|---|
| cPND1 | 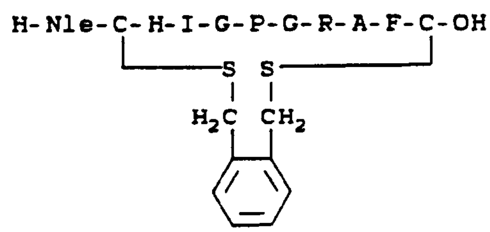 |
| cPND2 | 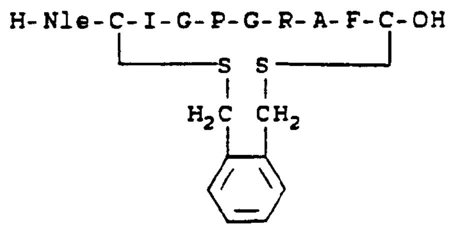 |
| cPND3 | 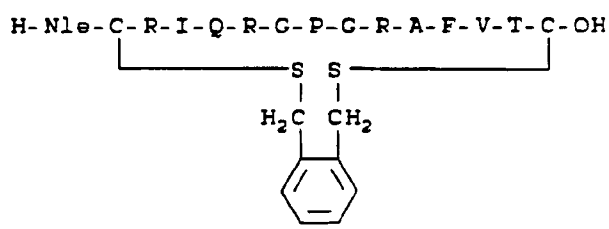 |

c. Cyclization through Amide bond Formation:

Novel amide bonded cyclic HIV PND peptides may be prepared for conjugation in essentially two phases: First, the linear peptide is prepared, for example on an ABI-431A peptide synthesizer, by known solid phase peptide synthetic chemistry, for example using Fmoc chemistry and appropriately side-chain protected Fmoc-amino acids as reagents.

Second, the linear peptide is cleaved from the resin and cyclized in solution by allowing the free amino terminus of the peptide, the free amino group of an amino terminal isoglutamine, or a free ε-amino or α-amino group of a lysine on one side of the loop amino acids to be amide bonded to a free carboxyl group on the carboxy-terminal side of the loop amino acids through DPPA, BOP, or similar reagent mediated peptide bond formation.

Peptides synthesized according to this subsection are given below in Examples 6-26. Products obtained may be characterized by fast atom bombardment-mass spectrometry [FAB-MS], reverse phase HPLC, amino acid analysis, or nuclear magnetic resonance spectroscopy (NMR).

Thus, a highly preferred embodiment of the peptide-polysaccharide-protein conjugate consists essentially

of OMPC covalently coupled to an HIV PND peptide through a spacer comprising PRP or PnPs6B. Where the PND is from a predominant isolate, such as the HIV IIIB or the HIV MN isolate, a conjugate vaccine, or a mixture of such conjugate vaccines is highly advantageous for prophylaxis or treatment of AIDS or ARC. Examples of such preferred embodiments have the structure:

a) 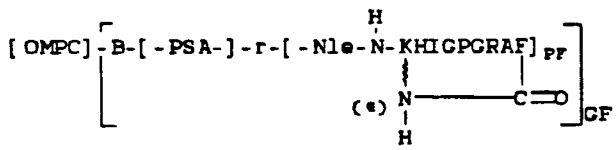 ,

b) 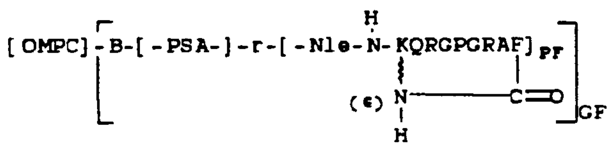 ,

c) 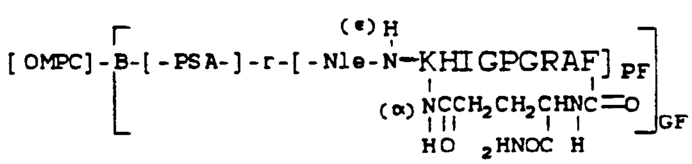 , or

d) 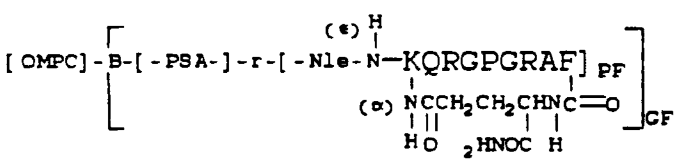 ;

wherein:
-r- and -B- are selected from:

a) $-CONH(CH_2)_4NHCOCH_2SCH_2CH(NHCOCH_3)CO-$,

b) $-CONH(CH_2)_4NHCOCH_2SCH_2CH_2CH(NHCOCH_3)CO-$, or

c) $-CONH(CH_2)_2SCH_2CO-$;

PSA is comprised of either PRP or PnPs6B;

PF indicates a mass ratio of between 0.01 and 1 milligrams of peptide per milligram of PRP or PnPs6B; and

GF indicates a mass ratio of between 0.01 and 0.33 milligrams of PRP or PnPs6B per milligram of OMPC.

The following examples are provided to more particularly demonstrate how to make and use the conjugate of this invention. However, the examples provided are not to be construed so as to limit the scope of the invention.

A. Examples of Peptide Preparations:

EXAMPLE 1

Solution Cyclization to Form cPND1:

Anhydrous DMF (20 ml) was degassed and HPLC-isolated linear 11-mer H-NleCHIGPGRAFC-OH (20 mg, 17 μmole) was dissolved and sealed under a nitrogen atmosphere. A solution of o-xylylene dibromide (4.7 mg, 17.9 μmole) in anhydrous DMF (50 μl) was added. Next, $NEt_3$ (11.9 μl, 85.2 μmole) in DMF was added over a

period of about 6.5 hours. About one hour after complete addition of the base, the solution was dried. The residue was resuspended in ether, centrifuged to spin down the insoluble product, and then redried. An aliquot analyzed by fast atom bombardment-mass spectrometry (FAB-MS) yielded a major ion $[M+H]^+$ of 1275. Isocratic reversed-phase HPLC on a Vydac C18 column (0.46 x 25.0 cm) using 0.1% TFA/24% $CH_3CN$ at 2.0 ml-/minute, monitored at 215 nm, showed a sharp product peak having a retention time of about 18.5 minutes. A preparative scale isolation was run over 135 minutes at 10 ml/minute from 24-29% $CH_3CN$, and the product with a retention time of 76.41 minutes under these conditions was collected and rechromatographed under analytical conditions to confirm its identity. Amino acid analysis and 400 MHz NMR analyses were consistent with the structure cPND1:

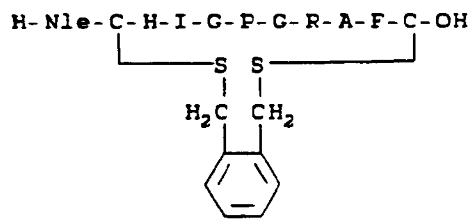

EXAMPLE 2

Solution Cyclization to Form cPND2:

The procedure described in Example 1 was followed except that the linear 10-mer H-Nle-CIGPGRAFC-OH (20 mg, 19.3 μmole) was used. After the addition of base, the solution was kept under nitrogen for an additional 9 hours. FAB-MS yielded $[M+H]^+ = 1138$ and $[M+Na]^+ = 1160$, consistent with the structure proposed for cPND2. Preparative HPLC using two 2.12 x 25 cm Vydac C18 columns was conducted over 90 minutes from 24% to 28 % $CH_3CN$/0.1% TFA, at 10 ml/minute. The eluate was monitored at 215 nm and two product peaks at retention times of 63.42′ and 70.82′ were collected, dried, and sujected to FAB-MS. Both materials had $[M+H)^+$ of 1138, (the later peak also had a $[M+Na)^+$ of 1160) which is consistent with the structure cPND2:

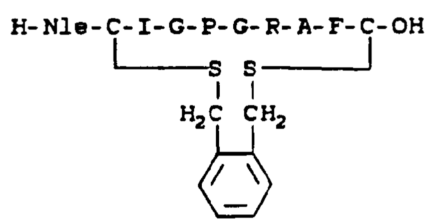

EXAMPLE 3

Solution Cyclization to Form cPND3:

The same procedure used in Examples 1 and 2 was employed here except that the linear 15-mer H-Nle-CRIQRGPGRAFVTC-OH (21.2 mg, 11.92 μmole) was used, and $NEt_3$ was added over about 10 hours. An additional 5-hour exposure to base was permitted prior to analysis by analytical HPLC. FAB-MS of the crude product showed a single intense $[M+H]^+$ of 1779 which is consistent with the structure of cPND3:

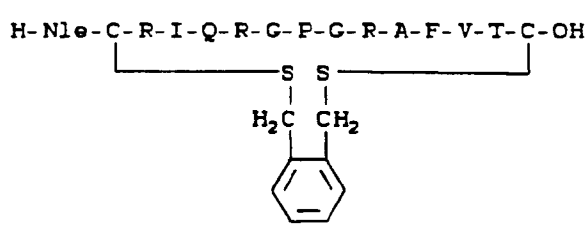

EXAMPLE 4

Solid State Cyclization to Form cPND1:

A linear PND peptide was prepared on Wang resin on an ABI-431A peptide synthesizer, starting from Fmoc-L-Cys(Acm)-O-Wang resin (0.61 meq/gram). Fmoc chemistry and Fmoc-amino acid symmetrical anhydrides (4x excess, prepared in situ) were used as reagents on a 0.25 mmole scale to generate 745 mg of the peptide:

```
              Acm           Mtr
Fmoc-Nle-C-H-I-G-P-G-R-A-F-C-O-Wang-resin.
              Trt               Acm
```

$Hg(OAC)_2$ (64 mg, 0.2 mmole) was dissolved in 10% acetic acid in DMF (0.5 ml) and added to the dried resin (149 mg, 0.05 meq) shown above. More 10% acetic acid in DMF (0.2 ml) was added to the swollen resin, and the solution was stirred overnight. Thereafter the resin was filtered, washed with DMF (5 x 1 ml), $CH_2Cl_2$ (3 x 1 ml), and ether (3 x 2 ml). Subsequently, the resin was dried and 1 ml $H_2S$ saturated DMF was added, followed by a second aliquot of the same. The resin was then filtered and washed as above and then dried, yielding a black resinous powder (179 mg).

A mixture of o-xylylene dibromide (3.2 mg) and $NEt_3$ (3.4 μl) in DMF (2 ml) was added to the resin (35 mg) at room temperature, and allowed to react for 16 hours. The resin was filtered, washed as above, and dried. The Fmoc was removed by treatment with 20% piperidine in DMF (1 ml) over 20 minutes at room temperature. The resin was washed again as above, and dried.

The cPND1 peptide was cleaved from the resin, and Trt and Mtr protecting groups concomitantly removed, by treating with 95% TFA/4% ethane dithiol/1% thioanisole (1 ml) at room temperature over 6 hours. The solution was filtered, the resin washed with additional 100% TFA (3 x 1 ml), and the combined filtrate was evaporated at 20°C/0.1 mm pressure. Material that was insoluble in ether was removed by extraction (3 x 2 ml) and the insoluble crude product was redried at 20°C/0.1mm pressure.

Analytical HPLC using a 0.46 x 25 cm Vydac C18 column was used to identify the product. Comparison with the product obtained from Example 1 confirmed that authentic product was present (retention time of 12.88′ as compared with 12.97′). Preparative HPLC was conducted over 90 minutes from 25% to 30% $CH_3CN$/0.1% TFA at 10 ml/minute using two 2.12 x 25 cm Vydac C18 columns in series. The peak eluting at 54.12′ was collected. Co-chromatography, on an analytical scale, of this material with material prepared in Example 1 showed a single sharp peak. FAB-MS had a $[M+H]^+$ of 1275, confirming the preparation of cPND1 (see Example 1 above for structure).

EXAMPLE 5

Solid State Synthesis of Disulfide-Bonded cPND4:

A linear PND peptide was prepared on Wang resin using an ABI-431A peptide synthesizer, starting from Fmoc-L-Cys(Acm)-O-Wang resin (0.61 meq/gram). Fmoc chemistry and Fmoc-Amino Acid symmetrical anhydrides (4X excess, prepared in situ) were used as reagents on a 0.25 mmole scale to generate 745 mg of the peptide:

```
              Acm           Mtr
Fmoc-Nle-C-H-I-G-P-G-R-A-F-C-O-Wang-resin.
              Trt               Acm
```

A solution of iodine in 5% methanol/anhydrous DMF (1 ml) was added to the dried, derivatized Wang resin shown above and stirred at room temperature for 4 hours. The resin was filtered, washed with anhydrous DMF (5 x 2 ml), and finally resuspended in DMF (2 ml). Two drops of a 0.1 M solution of sodium thiosulphate in water were added, and stirred for a few seconds. The resin was washed with aqueous 95% DMF (3 x 2 ml), anhydrous DMF (2 ml), methylene chloride (3 x 2 ml), ether (3 x 2 ml) and dried.

The Fmoc and other protecting groups were removed by treatment with 20% piperidine in DMF over 20 minutes, and the resin was washed and dried. The resin was cleaved from the disulfide bonded cyclic peptide

by treatment with 95% TFA/4% ethane dithiol/1% thioanisole (1 ml) at room temperature for 6 hours. The solution was filtered, the resin washed with additional 100% TFA (3 x 1 ml), and the combined filtrate dried. Material that was insoluble in ether was removed by extraction (3 x 2 ml) and the solution redried.

Preparative HPLC using two 2.12 x 25 cm Vydac C18 reverse phase columns in series and a gradient elution of 20 to 24% $CH_3CN$ over 90′ allowed isolation of a sharp peak eluting at 36.66′ under these conditions. Analytical HPLC yielded a single peak upon co-chromatography of a known disulfide bonded cyclic standard with the product obtained from preparative HPLC. FAB-MS gave a $[M+H]^+$ of 1171, which is consistent with the the disulfide bonded cyclic structure cPND4:

H-Nle-CHIGPGRAFC-COOH
$CH_2$—S—S—$CH_2$

EXAMPLE 5-b

Synthesis od Disulfide Bonded CPND33:

1. SYNTHESIS OF: H-Nle Cys Tyr Asn Lys Arg Lys Arg Ile His Ile Gly Pro Gly Arg Ala Phe Tyr Thr Thr Lys Asn Ile Ile Gly Cys-OH ($C_{135}H_{220}N_{42}O_{33}S_2$, formula weight = 3023.6)

The 26mer was assembled on the Milligen # 9050 synthesizer, starting from the partially racemised Fmoc-L-Cys(Trt)-OPKA resin (Milligen batch B 090426) using 0.081 meq/g (about 604 mg), using 2.47 g (0.200 meq). The resin was mixed with an equal volume of glass beads (Sigma 150-212 μm). The mixture completely filled two 1 x 10 ccm columns, connected in series. Reagents were Fmoc-Pft ester (except for threonine, which was dHBt), using in four fold molar excess in N-methyl pyrrolidine solvent. Side chain protection was: Y 9tert-butyl); K (Boc); R (Mtr); His (Boc; T (tert-butyl); C (Trt). The protocol was modified to give double coupling with $K^7$; $I^9$; $I^{11}$; $G^{12}$; $P^{13}$; $G^{14}$; $R^{15}$; $F^{17}$; $Y^{18}$; $T^{19}$; $T^{20}$;$I^{23}$; $I^{24}$. Acylation recycle times were extended from 30 to 60 minutes for all units, except for $G^{14}$ and $A^{16}$, and to 90 minutes for $I^9$ (2x); $I^{11}$ (2x); $I^{23}$ (2x) and $I^{24}$ (2x). The derivatized resin was maintained as the free terminal amine which was washed with $CH_2Cl_2$ and air-dried.

The mixture of dry derivatized resin and glass beads was resuspended in 95% TFA, 4% ethane dithiol, 1% $CH_3Sph$ (30 mL) at 23°C in a sealed flask, with gentle stirring on an oscillating tray for 8 hours. The bright yellow mixture was then filtered and the insolubles were thoroughly extracted with 100% TFA (3 x 20 mL). The combined dark orange filtrates were evaporated to give a pale tan, oily gum. On trituration with ether (20 mL) this material instantly became a colorless solid, which was transferred to a filter by triturating with additional ether (3 x 20 mL). After drying, the crude product was obtained as a fine colorless powder (583 mg).

Analytical reverse phase HPLC on a 0.46 x 25.0 cm Vydac $C_{18}$ column of about a 50 μg sample, dissolved in aqueous 0.1% TFA/20% $CH_3CN$, revealed a major component and a later eluting minor component. These were separately collected after injection of a 30 mg and another 50 mg aliquot of the sample onto two 2.21 x 25.0 cm preparative columns in series. A total of 35.2 mg of the earlier eluting material and 8.2 mg of the later eluting material was recovered following lyophilization. FAB-MS gave a [M+H]+ = 3022.1 and an [M+Na] = 3044.2, which correlates with the calculated mass.

2. PREPARATION OF THE CYCLIC DISULFIDE:

H-Nle Cys Tyr Asn Lys Arg Lys Arg Ile His Ile Gly Pro
Gly Arg Ala Phe Tyr Thr Thr Lys Asn
$CH_2$ Ile Ile Gly Cys-OH
S —————————— S—$CH_2$

(SEQ ID: 1:)

a. K3Fe(CN)6 INDUCED OXIDATION:

The linear 26 mer dithiol compound (35.0 mg) was dissolved in degassed distilled water (38 mL) at 23°C to give a clear colorless solution at pH 2.73. The pH was adjusted to 8.5 with 0.1 N $NH_4OH$, and the solution

was covered with an atmosphere of nitrogen. An aliquot of the material was immediatley run on analytical reverse phase HPLC and found to be undergoing oxidation as evidenced by the appearance of a early peak.

With magnetic stirring, a freshly prepared solution of 0.01 M $K_3Fe(CN)6$ was added by power driven hypodermic syringe at 23°C under nitrogen. Analysis of a small aliquot by HPLC revealed total conversion of starting material to an earlier elution time. The reaction mixture (pH 8.3) was mixed with 10% aqueous acetic acid and stirred to give a pH of 4.0. The solution was filtered to remove insoluble material, and the faintly yellow solution was evaporated and then lyophilized to give about 27.9 mg of a pale yellow powder. The material was dissolved in 0.1% TFA, 20% $CH_3CN$ and gradient eluted on a preparative HPLC. A major early eluting peak and a later eluting peak (4:1) were separately collected, lyophilized to yield 6.1 mg of the early and 1.5 mg of the late eluting materials. FAB-MS analysis of the early eluting material: [M+H]+ 3019.7; [M+Na]+ 3042.5; FAB-MS analysis of the late eluting material: [M+H]+ 3021.0; [M+Na]+ early material = 3041.5; all of which corresponds to the correct mass for the cyclized cPND33. The later eluting material is the D-cysteine isomer.

Amino acid analysis of the products gave the predicted amino acid compositions for the cyclized products and confirmed that the later eluting material is the D-cysteine containing diastereomer.

b. AIR OXIDATION:

The linear 26 mer prepared in (1) above (86 mg, 28.4 μmoles) was dissolved in aqueous 0.1% TFA, 20% acetonitrile (284 mL) at 23°C and the solution was allowed to stand open to the air.

Cyclization was monitored by reverse phase HPLC and the sample was found to be almost completely converted to the early eluting material, with almost complete dissappearance of starting linear material, by t = 24 hours. The clear, colorless solution was evaporated to about 8 mL at which point an additional 10 mg sample prepared in the same way as the 86 mg, was added. The combined sample was evaporated to about 9 mL. The cloudy colorless solution was subjected to HPLC separation, in two separate runs, on two 2.12 x 25.0 cm Vydac $C_{18}$ columns in series. Two peaks of material were separately collected, an early eluting peak and a later eluting peak. Each peak was separately evaporated and lyophilized to yield 30.1 mg and 9.7 mg of the early and late materials respectively. The early eluting material was combined with other preparations of early eluting cyclized material to yield a total of 47.5 mg of a faintly bluish fluffly powder. Analytical HPLC of this material gave a single peak.

EXAMPLE 6

Solution Synthesis of Peptide Bonded cPND7:

The linear peptide Cbz-Nle-Lys(Boc)-His(Trt)-Ile-Gly-Pro-Gly-Arg(Mtr)-Ala -Phe was synthesized following solid-phase methods on an ABI 431A peptide synthesizer using 373 milligrams (0.1 mmoles) of commercially available Fmoc-Phenylalanyl-p- alkoxybenzyl alcohol resin. With the exception of norleucine, which was purchased in the benzyloxycarbonyl (Cbz) protected form, L-amino acids used were the fluorenylmethoxycarbonyl (Fmoc) derivatives having the appropriate acid-labile side chain protecting groups. The polypeptide-derivatized resin product was transferred to a sintered glass funnel, washed with dichloromethane, and dried, to yield 0.6 g of polypeptide-resin product.

The peptide was cleaved from the resin by treatment with 6 ml of a 95:2:3 mixture of TFA:1,2 ethanediol:anisole for 16 hours. The reaction mixture was filtered through a sintered glass funnel, the resin washed with 10 ml TFA, and the filtrates combined. Following concentration to about 1 to 2 ml of yellow oil, the linear peptide was recovered by trituration with 400 ml of diethyl ether, in 50 ml portions, and filtration on a sintered glass funnel. Dissolution with 100 ml 1% TFA followed by lyophilization yielded 298 mg of linear peptide.

The peptide powder was dissolved in 800 ml DMF, neutralized with 0.42 ml diisopropylethylamine, and treated with 0.077 ml diphenylphosphorylazide. The solution was stirred in the dark for 70 hours at 4°C to allow formation of the cyclic lactam. After quenching by addition of 3 ml glacial acetic acid, the reaction mixture was concentrated to about 1 to 2 ml of oil, dissolved in 10% aqueous acetic acid, and lyophilized.

The cyclic peptide was purified by G-15 size exclusion chromatography using 5% acetic acid as the mobile phase. Fractions, monitored by UV detection, containing the peptide were pooled and lyophilized to yield 135 mg of dry cyclic peptide. All results obtained were consistent with the structure cPND7:

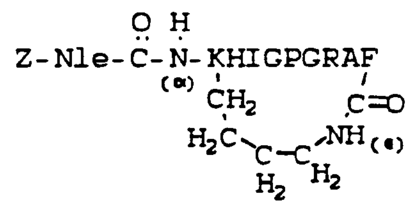

which may also be represented as:

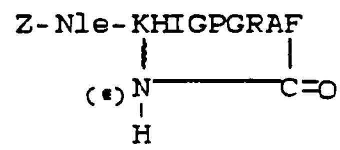

EXAMPLE 7

Deprotection of cPND7 to yield the hydrogen form, cPND8:

Deprotection of cPND7 was achieved by dissolving the cyclic peptide in 20 ml of 30% aqueous acetic acid and hydrogenation at 40 psi for 16 hours over 100 mg of 10% palladium on carbon. The reaction mixture was filtered over celite to remove the catalyst, and the filtrate was lyophilized. Reverse phase HPLC using a Vydac C18 semi-prep column was utilized to obtain 8.5 mg of pure deprotected cyclic peptide. This method of deprotection is applicable to all peptides synthesized as the benzyloxycarbonyl N-protected peptide, to yield the free hydrogen form of the peptide which may now be activated at the amino terminus in preparation for conjugation. The structure of the product was confirmed by FAB-MS, analytical HPLC and amino acid analysis, and all results were consistent with the structure cPND8:

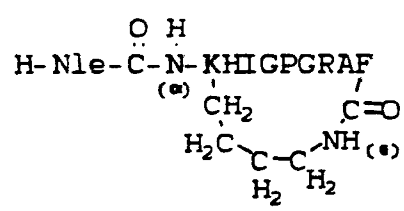

which may also be represented as:

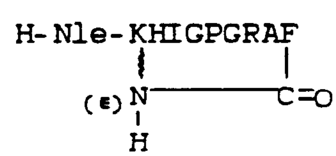

EXAMPLE 8

Synthesis of cPND10:

The synthesis of cPND10, having an Acm protected Ac-cysteine at the peptide amino-terminus, is identical to the procedure used in Example 1, except that the synthesis here included an Fmoc-norleucine, rather than the Z-Nle, and the additional amino acid, Ac-Cys(Acm) was used as the N-terminal amino acid. Thus, the linear peptide Ac-Cys(Acm)-Nle-Lys(Boc)-His(Trt)-Ile-Gly-Pro-Gly-Arg(Mtr)-Ala-Phe, was assembled using commercially available Fmoc-Nle, and Fmoc-Cys(Acm). This modification of the Example 1 procedure is applicable to the synthesis of other cPND peptides where an N-terminal Ac-Cys(Acm) is desirable.

EXAMPLE 9

Deprotection of cPND10 to yield cPND9:

The Acm protected Ac-Cys(Acm) may be converted to the free Ac-Cys-SH (free sulfhydryl) form of the peptide according to the procedure described in Atherton, E. et al., Chem. Soc. Perkin Trans., I, 2057 (1985). This procedure is applicable to removal of Acm thiol protection of peptides in preparation for conjugation with a thiophilic agent, such as bromoacetylated or maleimidated proteins or polysaccharides. A portion of cPND10 was dissolved in 10% aqueous acetic acid and treated with mercuric trifluoroacetate (10-fold excess). The pH was readjusted to 4 and the solution was stirred at room temperature while cleavage of the S-Acm groups was monitored by reverse-phase HPLC. When the reaction was judged completem, the solution was saturated with hydrogen sulfid gas. The mercury(II) sulfid precipitate was removed by centrufugation, and cPND9 was purified by RP-HPLC. The structure and purity of cPND9 was confirmed by FAB-MS, analytical HPLC, and amino acid analysis.

EXAMPLES 7-26

cPND Peptides Synthesized According to the Methods of Examples 6-9 and 19-20:

The procedures established above in Examples 6-9 and below in Examples 25-26 for the synthesis of cPND7, cPND8, cPND9, cPND10, cPND31, and cPND32 respectively were applied, without any substantial modification, aside from changes in the peptide primary sequence and inclusion of appropriate protecting groups, in the synthesis of the cyclic form of synthetic PND peptides from many different isolates. Thus, all of the following peptides, synthesized according to these methods, may be N-terminal deprotected as necessary, and conjugated through -r- to form the conjugates of this invention:

| EX. # | NAME | STRUCTURE | FAB MS M+H |
|---|---|---|---|
| 7) | cPND8 | 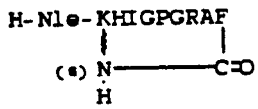 , | 1077 |
| 8) | cPND10 | 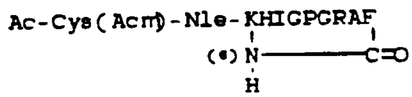 , | 1293 |
| 9) | cPND9 | 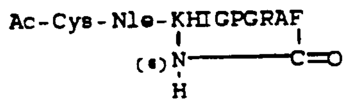 , | 1223 |
| 10) | cPND11 | 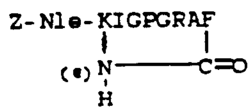 , | 1074 |
| 11) | cPND12 | 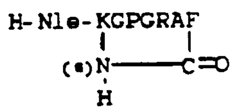 , | 827 |
| 12) | cPND13 | 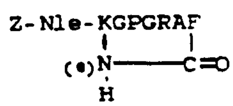 , | 961 |
| 13) | cPND14 | 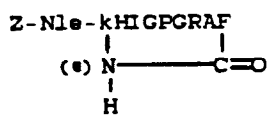 , | 1211 |
| 14) | cPND15 | 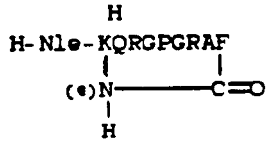 , | 1111 |

| EX. # | NAME | STRUCTURE | FAB-MS $M^+H$ |
|---|---|---|---|
| 15) | cPND16 | 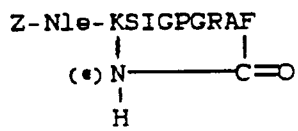 , | 1161 |
| 16) | cPND21 | 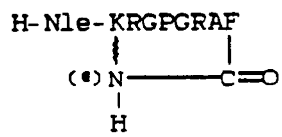 , | 983 |
| 17) | cPND23 | 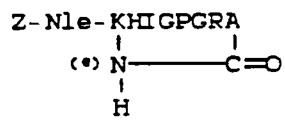 , | 1063 |
| 18) | cPND24 | 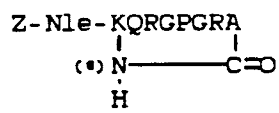 , | 1098 |
| 19) | cPND25 | 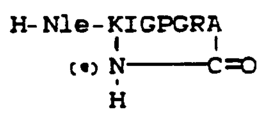 , | |

| Ex. # | Name | Structure | FAB-MS M+H |
|---|---|---|---|
| 20) | cPND26 | 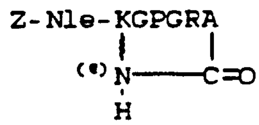 , | 814 |
| 21) | cPND27 | 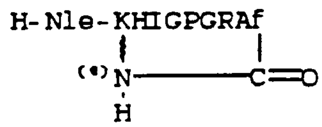 , | 1077 |
| 22) | cPND28 | 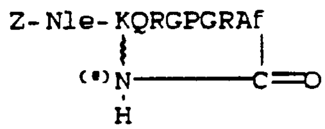 , | 1245 |
| 23) | cPND29 | 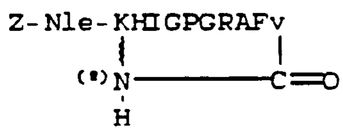 , | 1310 |
| 24) | cPND30 | 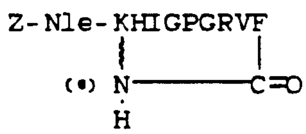 , | 1257 |
| 25) | cPND31 | 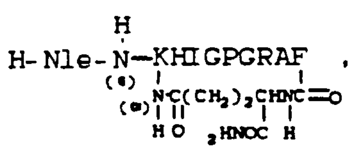 , | 1204 |
| 26) | cPND32 | 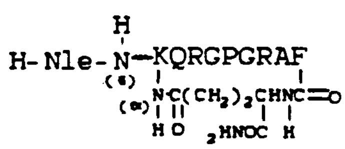 . | 1238 |

EXAMPLE 25

Synthesis of cPND31:

Two grams (0.6 meq/gram) of Fmoc-Phe-Wang resin was loaded on an ABI 431A synthesizer. Fmoc single coupling protocols were used to add Fmoc-Ala, Fmoc-Arg(Tos), Fmoc-Pro, Fmoc-Ile, Fmoc-His(Trt), Boc-Lys-(Fmoc), and Cbz-Nle to produce 3.7 grams of linear peptide resin having the sequence: Boc-Lys(N$^{\varepsilon}$-Z-Nle)-His(Trt)-Ile-Gly-Pro-Gly-Arg(Tos)-Ala-Phe.

The peptide was cleaved from the resin by treating with 95% TFA, 5% water for two hours. The resin was removed by filtration, the TFA removed from the filtrate by evaporation in vacuo, and the residue was triturated with diethyl ether. The precipitate was recovered by filtration and drying to yield 1.7 grams of linear peptide having the sequence:

H-Lys(N$^{\varepsilon}$-Z-Nle)-His-Ile-Gly-Pro-Gly-Arg(Tos)-Ala-Phe.

The peptide was treated with Boc-isoglutamine-ONp (0.71 grams, 2 nmoles,) and DIEA (0.35 ml, 2 mmoles) in DMF (10 ml) overnight at room temperature. The DMF was evaporated, and the residue treated with diethyl ether. The precipitate was recovered by filtration and washed with ethyl acetate. The dried peptide (1.9 grams) was treated with TFA (100 ml) for 0.5 hours. The TFA was evaporated in vacuo, the residue triturated with diethyl ether and the precipitate was recovered by filtration and dried.

The peptide was desalted on Sephadex G-10 in 10% aqueous acetic acid as the eluent. Peptide fractions were lyophilized to yield 1.2 grams (0.79 mmoles) of:

H-isoGln-Lys(N$^{\varepsilon}$-Z-Nle)-His-Ile-Gly- Pro-Gly-Arg(Tos)-Ala-Phe

Two batches (0.55 gm, 0.36 mmoles) of the peptide were separately dissolved in 1000 mL ice cold DMF and DIEA (0.16 mL, 0.9 mmoles) and DPPA (0.12 mL were added and the solutions were stirred overnight at room temperature. The DMF was evaporated in vacuo and the residues combined and solubilized in $CHCl_3$. The organic fraction was washed with 5% aqueous citric acid, then dried over $MgSo_4$ and evaporated to yield 0.78 gm of crude cyclic peptide. This material was treated with liquid HF (10 mL) containing anisole (1 mL) for two hours at 0°C. The HF was evaporated and the residue was purified by graidien elution on reveresed phase HPLC (Vydac C-18, 0-50% $CH_3CN$, over 50 minutes using 0.1 % aqueous TFA as the buffer) to give 250 mg of pure cPND31 (M+H=1204).

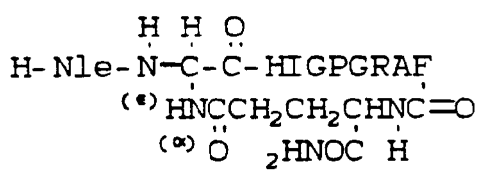

EXAMPLE 26

Synthesis of cPND32:

Essentially the same procedure used in Example 20 for synthesis of cPND 31 was employed here except that the linear peptide that was cyclized had the sequence:

H-isoGln-Lys(N$^{\varepsilon}$-Z-Nle)-Gln-Arg(Tos)-Gly-Pro-Gly-Arg(Tos)-Ala-Phe.

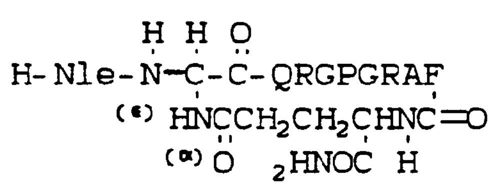

B. Examples of Intermediate Activation, Conjugate Formation and Immunological Analysis:

EXAMPLE 27

Preparation of the N-(bromoacetyl)-6-aminocaproic derivative of OMPC. (Brac-6-ACA-OMPC):

An OMPC solution (10 ml) (59 mg/ml) was centrifuged at 43K rpm, 4°c for 2 hours. The pellet was resuspended using a Dounce homogenizer in 6 ml of pH9 (Kolthoff) buffer and 1 milliliter of a N-(bromoacetyl)-6-aminocaproic acid p-nitrophenyl ester solution (85 mg/ml of acetonitrile) was added. The resultant mixture was agitated for 45 hours. Insoluble material was pelleted by a low speed centrifugation and the supernatant was then centrifuged at 43K rpm, 4°C for 2 hours. The pellet was resuspended on 10 ml of $H_2O$ and recentrifuged at 43K, 2 hours at 4°C. This pellet was resuspended in 10 ml of $H_2O$ affording the BrAc-6-ACA-OMPC. The amino acid analysis showed 268 nanomole/ml of -6-aminocaproic acid and 196 nanomoles/ml of lysine. The protein concentration was 2.4 mg/ml (41% recovery). To assay bromoacetylating potential, 1 ml of this solution was incubated at pH 8 with 30 μl of N-acetylcysteamine. After dialysis to remove excess reagent, the solvent was evaporated to yield the title compound. An aliqout of the sample was acid hydrolyzed and assayed by AA assay, revealing 54 nanomoles/ml S-carboxymethyl cysteamine and 140 namomoles/ml lysine (SCMC/lys = 0.38). This indicates that 38% of the lysines present are bromoacetylating moieties.

EXAMPLE 28

Preparation of the cystamine (Bis-2-aminoethyl disulfide) derivative of PRP. (PRP-cys-$NH_2$):

The tetrabutyl ammonium salt of PRP (300 mg) was dissolved in 11 ml of dimethyl formamide (DMF). Subsequently, 30 mg of carbonyldiimidazole was added and the solution stirred at room temperature (r.t.) for 1 hour. The DMF solution was then added with stirring to a chilled (ice) solution of cystamine dihydrochloride (450 mg/10ml $H_2O$; pH adjusted to 10.3 with NaOH) and stirring continued for 15 min. in the ice bath. The solution was then removed from the ice bath and aged at room temperature for 45 min. after transfer to a dialysis bag. Dialysis against (vs.) successive buffer changes was then effected as follows:

(a) vs. 4L pH 7, 0.1M $PO_4$ for 4.2 hours
(b) vs. 4L pH 7, 0.01M $PO_4$ for 8 hours;
(c) vs. 4L pH 7, 0.01M $PO_4$ for 16 hours and
(d) vs. 16L $H_2O$ for 8 hours.

Lyophilization afforded 150 mg of the title compound, cystamine derivative of PRP. The NMR of this material confirmed the cystamine derivatization of PRP. By comparing the two methylene resonances centered at 3 ppm ($CH_2$-S) with the glycosidic proton (5.1 ppm) or the total PRP proton integral, an average value of 48 cystamine moieties per 100 ribosyl-ribitol phosphate moieties (i.e. PRP monomeric unit) was calculated.

EXAMPLE 29

Reduction of PRP-cys-$NH_2$ to Yield PRP-SH:

To 9.0 ml of a pH 8.0 buffer (0.01M in $PO_4$, 0.005M in EDTA, = buffer A) was added 40 mg of PRP-cys-$NH_2$ (see Example 28 for preparation). After complete dissolution (15 min), 42 mg of solid dithiothreitol (DTT) was added. The mixture was degassed, nitrogenated, and aged for 4.5 hours at room temperature. The solution was then transferred to dialysis tubing and dialyzed against the buffer changes as follows:

1) vs. 4L buffer A for 16 hours;
2) vs. 4L buffer A for 7.25 hours;
3) vs. 1L of a pH 8, 0.1M $PO_4$ buffer, 0.005M in EDTA (buffer B) for 17 hours.

At this point an Ellman assay of the solution indicated a thiol titer of 2.04 mmoles SH/ml and therefore a total of 15.3 mmoles of the title PRP-SH for a volume of 7.5 ml.

EXAMPLE 30

Reaction of PRP-SH with bromoacetylated PND142 (BrAc-YNKRKRIHIGPGRAFYTTKNIIGT-OH) to yield PND142-PRP-SH:

To the PRP-SH solution prepared in EXAMPLE 29, was added 15.3 mg bromoacetylated PND142 (molecular weight of tris trifluoroacetate salt = 3268, estimated 4.7 μmoles, bromoacetylated by addition of N-(bromoacetyl)-6-aminocaproic acid p-nitrophenyl ester to the peptide and removal of the reagent by re-isolation of bromoacetylated peptide through reverse phase HPLC). The mixture was degassed and shaken for 6.25 hours. The SH titer by Ellman's assay at this point was diminished by only 1.2 μmoles. The pH was adjusted to 9.01 with 5 normal NaOH and the solution was allowed to age for an additional 16 hours. Ellman assay at this point indicated a loss of 7.9 μmoles of SH. 100 μl of the solution was assayed by the AA assay method and found to have 213 nanomoles of S-carboxymethyl cysteamine (SCMC) per ml (i.e. conjugated peptide). 100 μl was assayed for ribose and a concentration of 3.18 mg PRP/ml was determined. Thus, the title compound contains 67 nanomoles (0.067 mmoles) of peptide/mg of PRP or, using a m.w. of 2926, PF = 0.196 mg peptide/mg PRP.

EXAMPLE 31

Reaction of the PND142-PRP-SH with bromoacetylated OMPC to yield PND142-PRP-OMPC:

A solution of BrAc-ACA-OMP (5.5 ml, 2.4 mg protein/ml) was added to the peptidylated, thiolated PRP solution, PND142-PRP-SH, prepared in EXAMPLE 30. The pH was determined to be 7.98 and the solution was aged at room temperature for 1.5 hours under nitrogen. An Ellman assay at this point indicated that 4 μmoles of SH (of an initial total of 7.4 μmoles SH) remained. To "cap" these residual reactive sites, 4 mg of N-ethyl maleimide (NEM) was added and the solution aged for 21 hours. The solution was then transferred to a polycarbonate centrifuge tube, topped with $H_2O$ and centrifuged at 43,000 RPM for 2 hours at 4°C. The pellet was resuspended in 10 ml of $H_2O$ using a Dounce tissue homogenizer. After recentrifugation using the above conditions, the pellet was resuspended in 6.5 ml of $H_2O$. Lowry assay showed 1.4 mg of protein/ml and a ribose assay showed 62.6 μg PRP/ml, yielding a GF = 0.0447. Using this GF and the PF obtained in EXAMPLE 30, one obtains (PF X GF) = ELF = 0.0088 mg peptide/mg protein in the title compound.

EXAMPLE 32

Reaction of PRP-SH with bromoacetylated PND135 (BrAc-NNTRKSIRIQRGPGRAFVTIGKIGN-OH) to yield PND135-PRP-SH:

The reduction of PRP-cys-$NH_2$ was effected by the same procedure as for EXAMPLE 29. The Ellman titer indicated 4.2 μmoles SH/ml, yielding, in 8.7 ml a total of 36.6 μmoles SH.

Approximately 25.8 mg of bromoacetylated PND135 (7.5 μmoles, m.w.2875; estimated pentatrifluoroacetate, 3500) was added to the above thiolated PRP solution. The solution was degassed and aged for 8 hours. 100 μl of this solution was analyzed by AA assay and found to contain 0.300 μmoles of S-carboxymethyl- cysteamine/ml. A separate 100 μl assay indicated the presence of 3.3 mg PRP/ml. This affords a factor of 91 nanomoles peptide/mg PRP or using a m.w. of 2875, yields a 0.8625 mg PEP/ml. Thus, PF = 0.261 mg peptide/mg PRP in the title compound.

EXAMPLE 33

Reaction of the PND135-PRP-SH with bromoacetylated OMPC to yield PND135-PRP-OMPC:

The solution prepared in EXAMPLE 32 was sterile filtered through a 0.2 μm filter and then added to 12 ml of a BrAc-ACA-OMPC solution (2.7 mg protein/ml by Lowry assay). After degassing, the solution was aged for 44 hours and then capped by treating with 5 mg of NEM in 0.5 ml (pH 8) $PO_4$ buffer for 20 hours. The solution was centrifuged at 43K rpm, at 4°C for 2 hours. The pellet was resuspended in 20 ml of $H_2O$ and the centrifugation process was repeated. The final pellet was resuspended in 12 ml of $H_2O$. A Lowry protein assay showed 1.7 mg PRO/ml and a ribose assay showed 0.167 mg of PRP/ml, yielding a GF = 0.0982. Using this GF and the PF obtained in Example 32, the product (PF X GF) yields ELF = 0.0256 mg peptide/mg protein in the title compound.

EXAMPLE 34

Reaction of PRP-SH with bromoacetylated PND135-18 (BrAc-RIQRGPGRAFVTIGKIGN) to yield PND135-18-PRP-SH:

A PRP-SH solution (pH 8) was prepared as in Example 29 from 51 mg of PRP-cys-$NH_2$, affording 18.4

μmoles of SH (total) by Ellman assay. To this was added 18 mg of BrAc-RIQRGPGRAFVTIGKIGN-OH and the solution was aged for 18.5 hours. A small amount of precipitate was removed by a low speed centrifugation, and the solution was filtered through a 0.2 micron filter. This solution had a thiol titer of 3 μmoles SH (total), indicating a loss of about 15 μmoles of thiol. 100 μl of this solution was assayed for ribose resulting in a titer of 1.66 mg PRP/ml. Another 100 μl aliqout was assayed by AA analysis and a SCMC concentration of 0.195 μmoles/ml obtained. Thus a factor of 0.117 micromoles peptide/mg PRP was obtained. Using a molecular weight of 2061, a PF = 0.242 mg peptide/mg PRP was obtained.

EXAMPLE 35

Reaction of PND135-18-PRP-SH with bromoacetylated OMPC to yield PND135-18-PRP-OMPC:

To the filtered solution from Example 34 was added 4.5 ml of Br-ACA-OMPC (2.4 mg/ml). The solution was then degassed and aged for 52 hours at room temperature. Then 4.7 mg of NEM was added and ageing continued for a further 16 hours. The solution was then diluted to 20 ml with $H_2O$ and centrifuged for 2 hours at 4° C at 43,000 rpm. The pellet was resuspended in 10 ml of $H_2O$ and recentrifuged at 43,000 rpm for 2 hours at 4° C. The pellet was suspended in 6.5 ml of $H_2O$ and assayed for ribose and protein. Values of 0.115 mg/ml PRP and 0.873 mg/ml protein were obtained to yield a GF = 0.132 mg PRP/mg OMPC. Using a PF = 0.242 from Example 34, the factor (PF X GF) = ELF = 0.0319 mg PEP/mg OMPC was obtained.

EXAMPLE 36

Reaction of PRP-SH with bromoacetylated PND135-12 (BrAc-RIQRGPGRAFVT-OH) to yield PND135-12-PRP-SH:

A PRP-SH solution (pH 8) was prepared as in Example 29 starting with 16.5 mg PRP-cys-$NH_2$, affording a total of 14.4 μmoles of SH by Ellman assay. To this solution (about 5 ml) was added 11.2 mg bromoacetylated PND135-12, (BrAc-RIQRGPGRAFVT). The mixture was degassed and aged for 6 hours at room temperature. An Ellman assay at this point indicated 7.8 μmoles of SH remained. A 100 μl AA analysis assay indicated the presence of 0.146 μmoles of covalent peptide (SCMC)/ml. Using a molecular weight of 1,338 for the peptide, this value converts to 0.1953 mg PEP/ml. A ribose assay indicated the presence of 3.3 mg/ml of PRP, affording a PF = 0.0592 mg PEP/mg PRP in the title compound.

EXAMPLE 37

Reaction of PND135-12-PRP-SH with bromoacetylated OMPC to yield PND135-12-PRP-OMPC:

To the filtered solution from Example 36 was added 4.5 ml of Br-ACA-OMPC (pH 7.8). The solution was degassed and aged for 87 hours at room temperature, after which 6 mg of NEM was added and ageing continued for an additional 24 hours at room temperature. The mixture was then transferred to a centrifuge tube, topped with $H_2O$, and centrifuged for 2 hours at 43,000 rpm at 4° C. The pellet was resuspended in 5 ml of $H_2O$ and assayed for protein (Lowry), affording a value of 2.9 mg OMPC/ml. A ribose assay showed 0.202 mg/ml of PRP, allowing the calculation of GF = 0.0697 mg PRP/mg OMPC. Using this GF and the PF obtained in Example 36, the product (PF X GF) = ELF = 0.0041 mg PEP/mg OMPC was obtained for the title comound.

EXAMPLE 38

Protocol for Inoculation of Animals with the PND142-PRP-OMPC or PND135-PRP-OMPC Conjugates:

Alum was used as an adjuvant during the inoculation series. The inoculum was prepared by dissolving the PND142-PRP-OMPC or PND135-PRP-OMPC conjugate in physiologic saline at a final conjugate concentration of 300 μg/ml. Preformed alum (aluminum hydroxide gel) was added to the solution to a final level of 500 μg/ml aluminum. The conjugate was allowed to adsorb onto the alum gel for two hours at room temperature. Following adsorption, the gel with the conjugate was washed twice with physiologic saline and resuspended in saline to a protein concentration of 300 μg/ml.

African green monkeys were individually inoculated with three 300 μg doses of the PND142-PRP-OMPC or three 100 μg doses of the PND135-PRP-OMPC conjugate adsorbed onto alum. Each dose was injected intramuscularly. The doses were delivered one month apart (week 0, 4 and 8). The animals were bled at intervals of two weeks. Serum samples were prepared from each bleed to assay for the development of specific antibodies as described in the subsequent examples.

EXAMPLE 39

Analysis of Sera for Anti-Peptide IgG Antibodies:

Each serum sample was analyzed by enzyme-linked immunoadsorbent assay (ELISA). Polystyrene microtiter plates were coated with 0.5 µg per well of the synthetic peptide (not conjugated to PRP-OMPC) in phosphate-buffered physiological saline (PBS) at 4°C. Each well was then washed with PBS containing 0.05% TWEEN-20 (PBS-T). Test serum, diluted serially in PBS-T, was added to the peptide-containing wells and allowed to react with the adsorbed peptide for one hour at 36°C. After washing with PBS-T, alkaline phosphatase-conjugated goat anti-human IgG was added to the test wells and was allowed to react for one hour at 36°C. The wells were then washed extensively in PBS-T. Each well received 0.1% p-nitrophenyl phosphate in 10% diethanolamine, pH 9.8, containing 0.5 mM $MgCl_2{\cdot}6H_2O$. The ensuing reaction was allowed to proceed at room temperature for 30 minutes, at which time it was terminated by the addition of 3.0 N NaOH.

The greater the interaction of antibodies in the test serum with the peptide substrate, the greater is the amount of alkaline phosphatase bound onto the well. The phosphatase enzyme mediates the breakdown of p-nitrophenyl phosphate into a molecular substance which absorbs light at a wavelength of 405 nm. Hence, there exists a direct relationship between the absorbance at 405 nm of light at the end of the ELISA reaction and the amount of peptide-bound antibody.

All the monkeys inoculated with the PEP-PRP-OMPC (PND142-PRP-OMPC or PND135-PRP-OMPC) conjugate developed antibodies specifically capable of binding the peptide.

EXAMPLE 40

Analysis of Sera for Activity which Specifically Neutralizes HIV Infectivity:

Virus-neutralizing activity was determined with an assay described by Robertson et al., J. Virol. Methods 20: 195-202 (1988). The assay measures specific HIV-neutralizing activity in test serum. The assay is based on the observation that MT-4 cells, a human T-lymphoid cell line, are readily susceptible to infection with HIV and, after a period of virus replication, are killed as a result of the infection.

The test serum was treated at 56°C for 60 minutes prior to the assay. This treatment is required to eliminate non-specific inhibitors of HIV replication. Heat treated serum, serially diluted in RPMI-1640 cell culture medium, was mixed with a standard infection dose of HIV. The dose had been determined prior to the assay as containing the smallest quantity of virus required to kill all the MT-4 cells in the assay culture after a period of 7-8 days. The serum-virus mixture was allowed to interact for one hour at 37°C. It then was added to $1.0 \times 10^5$ MT-4 cells suspended in RPMI-1640 growth medium supplemented with 10% fetal bovine serum. The cultures were incubated at 37°C in a 5% $CO_2$ atmosphere for 7 days.

At the end of the incubation period, a metabolic dye, DTT, was added to each culture. This dye is yellow in color upon visual inspection. In the presence of live cells, the dye is metabolically processed to a molecular species which yields a blue visual color. Neutralized HIV cannot replicate in the target MT-4 cells and therefore does not kill the cells. Hence, positive neutralization is assessed by the development of blue color following addition of the metabolic dye.

All the monkeys inoculated with the PND142-PRP-OMPC or PND135-PRP-OMPC conjugate developed specific HIV infectivity-neutralizing activity.

EXAMPLE 41

Preparation of cPND9-PnPs6B-OMPC (cPND9 =

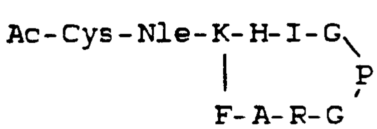

):

PnPs6B(n-Bu4N+):

PnPs6B (484 mg) was dissolved in distilled water (48 mL) and the solution magnetically stirred until all solids went into solution (1.5 h). While the polysaccharide was dissolving, a column (20 mm) was charged with Dowex 50 X 2 ($n\text{-}Bu_4N^+$) ion exchange resin (30 mL) and the resin bed washed with water for 1.5 hr. The viscous polysaccharide solution was applied to the rinsed resin and allowed to pass through the bed by gravity (16 h). The column was washed with water (8 mL) and the combined eluants lyophilized, providing 800 mg of a pale yellow solid that was dried in a vacuum desiccator (50 mm Hg, 60 h) over $P_2O_5$. This procedure provided 564 mg of dry PnPs6B tetra-n-butyl ammonium salt, PnPs6B(n-Bu4N+).

PnPs6B-$BuA_2$:

PnPs6B($n\text{-}Bu_4N^+$)(140 mg) was dissolved in dimethylsulfoxide (5 mL) and magnetically stirred for 15 min, at which time all solids appeared to be in solution. To this mixture was added 1, 1′-carbonyldiimidazole (15 mg), in one portion, and the reaction stirred at room temperature (80 min). In a separate flask, a solution of butanediamine dihydrochloride ($BuA_2\cdot 2HCl$, 344 mg) in water (5 mL) was made basic (pH 10.2) by the addition of 2.5 N NaOH then cooled to 0°C. To this cold mixture was added the activated polysaccharide, in a slow steady stream, and the resulting solution stirred at 0°C (30 min). The reaction mixture was allowed to warm up to room temperature and stirred for an additional 1 h, after which it was transferred to dialysis tubing (2 mL/cm) and dialyzed (4°C) against the following: 4 L 0.1 M pH 7.0 $HPO_4$ buffer, 5 h; 4 L 0.01 M pH 7.0 $HPO_4$ buffer, 15 h; 4 L 0.01 M pH 7.0 $HPO_4$ buffer, 9 h; and 4 L distilled $H_2O$, 15 h. The contents of the dialysis tubing were lyophilized, providing a white solid that was dried overnight (16 h) in a desiccator over $P_2O_5$, to yield 94 mg of PnPs6B-Butane diamine (PnPs6B-$BuN_2$). The NMR (300 MHz, $D_2O$) of this material revealed that a 50% loading (50 $BuA_2$ units per 100 6B monomer units) of butanediamine was achieved. The percent loading was determined by comparing the integration of the butanediamine methylene protons to rhamnose methyl protons.

PnPs6B-BiA2-BrAc:

6B-$BuA_2$ (94 mg) was dissolved in pH 9.04 Kolthoff buffer (10 mL) and the mixture magnetically stirred for 30 min to effect solution. To this aqueous solution was added a mixture consisting of p-nitrophenyl bromoacetate (94 mg) in acetonitrile (1 mL) and the reation stirred overnight (22 h) in the cold-room (4° C). The resulting viscous yellow solution was transferred to dialysis tubing (2 mL/cm) and dialyzed (4°C) against the following: 15 L distilled $H_2O$, 23 h; and 4L distilled $H_2O$, 23 h. The contents of the dialysis bag were stored as a solution. One mL of this solution was lyophilized, providing 3.5 mg of PnPs6B-$BuA_2$-BrAc as a white solid. The NMR (300 MHz, $D_2O$) of this material showed that the loading of butanediamine was 28%. The discrepancy between this value and the one obtained earlier (on PnPs6B-$BuA_2$) may be due to a solubility problem since not all of the lyophilized material would go back into solution.

PnPs6B-cPND9:

Aqueous PnPs6B-$BuA_2$-BrAc (11 mL, approx. 38.5 mg) was added to a vial containing the cyclic peptide

cPND9 (cPND9 = 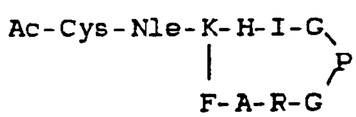 )

(8.5 mg, 6.9 pool, molecular weight of 1221). An Ellman's test was immediately performed on the resulting solution (200 μL sample) and showed an SH titer ($OD_{412}$=0.270) of 3.3 μmole. The reaction mixture was transferred to a 15 mL centrifuge tube, degassed and blanketed with nitrogen then tumbled at room temperature (5 h). The reaction mixture was transferred to dialysis tubing (2 mL/cm) and dialyzed against 4L distilled water (4°C, 23 h). One mL of the solution contained within the dialysis tubing was removed and lyophilized. The remaining (13.5 mL) solution's pH was adjusted to pH 8.3 by the addition of dried pH 8.0 $HPO_4$ buffer salt (180 mg) and a few drops of 5 N NaOH. This pH-adjusted solution was used for conjugation with thiolated OMPC.

cPND9-PnPs6B-OMPC:

Sterile OMPC (10 mL, approx. 45 mg) was pelleted down by ultracentrifugation (4°C to 20°C, 43 K rpm, 1.5 h). The pellet was resuspended in 6 mL of a sterile-filtered (Millipore Millex-GV 0.22 μm sterile filter) thiolation mixture which consisted of the following: homocysteinethiolactone hydrochloride (86 mg), ethylenediaminetetraacetic acid disodium salt (86 mg), and dithiothreitol (17 mg) in pH 11 borate buffer (10 mL). The pellet was homogenized (Dounce) and transferred to a sterile 15 mL centrifuge tube, degassed and blanketed with nitrogen. The reaction mixture was tumbled overnight (20.5 h) at room temperature, then transferred to an ultracentrifuge tube and topped with 1 M $KH_2PO_4$. The protein was pelleted down (4°C, 43 K rpm, 2 h), resuspended in 0.1 M $HPO_4$ buffer (10 mL), homogenized (Dounce) and repelleted (4°C, 43 K rpm, 2 h). The sterile protein pellet was used directly in the conjugation with the peptide-polysaccharide solution.

The sterile protein pellet was resuspended in sterile-filtered (Millipore Millex-GV 0.22 μm sterile filter) polysaccharide-peptide solution and homogenized (Dounce). An Ellman's test was performed immediately, and showed an SH titre of 15.3 μmol ($OD_{412}$=0.730). The reaction mixture was transferred to a sterile 15 mL centrifuge tube, degassed, blanketed with nitrogen, and aged overnight (21 h). An Ellman's test showed an SH titre of 8.2 μmol ($OD_{412}$=0.390). The protein was capped by the addition of 1 mL of a sterile-filtered (Millipore Millex-GV 0.22 μm sterile filter) solution consisting of the following: N-ethylmaleimide (75 mg) in pH 8.0 $HPO_4$ buffer (5 mL). This mixture was aged overnight (16 h) at room temperature. An Ellman's test was now visibly negative ($OD_{412}$=0.066, SH titer = 1.38 μmol). The sterile capped conjugate was pelleted by ultracentrifugation (4°C, 43 K rpm, 2 h), then resuspended and homogenized (Dounce) in sterile $H_2O$ (10 mL), and stored in a sterile plastic 15 mL centrifuge tube. The following assays were performed (amount solution used): Lowry protein, 1.50 mg/mL (100 μL); amino acid analysis, Nle= 2 nmoles/100 μL (100 μL). ELF may be directly calculated from these values as the Nle concentration is the same as the peptide concentration using a conversion of 1221 for the peptide molecular weight. Thus, the peptide concentration was calculated to be 0.0242 mg/ml, yielding an ELF of 0.0161 mg PEP/mg OMPC for the title conjugate.

EXAMPLE 42

Preparation of the cPND9-PRP-OMPC (cPND9 =

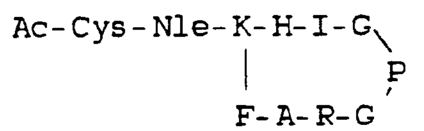

):

33 mg of PRP-$BuA_2$-BrAc (17 $BuA_2$-BrAc/100 PRP monomer units), prepared in the same manner as was the PnPs6B-$BuA_2$-BrAc in Example 46, was dissolved in 3.5 mL pH8 (0.1M $PO_4$) buffer. The solution was degassed and the air exchanged for $N_2$, and 6.9 mg of cPND9 added. If pure, this corresponds with 5.65 mmoles of cyclic peptide (M.W. 1221). However, an Ellman assay indicated the presence of only 1.5 μmoles of SH. The peptide-PRP solution was aged for 7 hr (Ellman=0) and then dialyzed vs. 4L of $H_2O$ for 16 hr. Freeze drying an aliquot afforded an NMR spectrum having phenyl resonances (7.28 and 7.43 ppm) diagnostic for the presence of peptide conjugated to the PRP.

OMPC was functionalized as usual with N-acetyl homocysteine thiolactone, affording a thiolated OMPC (7.35 μmoles SH/45 mg OMPC starting OMPC = 0.163 μmole SH/mg). The thiolated protein was resuspended in 3 mL of the peptidylated PRP which was first buffered to pH 8 with a phosphate salt and then filtered through a 0.22 μ sterile millex GV filter. This OMPC-peptidylated PRP mixture was degassed and aged for 41 hrs. At this time, about 55% of the thiol titer remained. A sterile filtered solution (0.6 mL) of N-ethylmaleimide (31 mg/3.5 mL) in pH 8 buffer was added and the reaction mixture aged for 2.5 hrs. It was then free of thiol. The solution was diluted to 10 mL with $H_2O$ and centrifuged at 43,000 rpm, for 2 hr at 4°C. The pellet was resuspended in 10 mL of $H_2O$ and recentrifuged as above. The pellet from the second centrifugation was resuspended in 7 mL of $H_2O$ and aged 60 hr at 4°C. A low speed (clinical spin) pelleted a small amount of precipitate. The supernatant was assayed by AA analysis, revealing a SCMHC/lys = 0.055, and a norleucine (Nle) concentration of 4.8 nanomole/ml, which translates into a peptide concentration of 0.0056 mg PEP/mL (using 1221 as the molecular weight for the peptide). Protein (Lowry) assay showed the presence of 0.810 mg/mL. Thus, the ELF = 0.0072 for the title conjugate.

While the foregoing specification teaches the principles of the present invention, with examples provided for the purpose of illustration, it will be understood that the practice of the invention encompasses all the usual

variations, adaptations, modifications, or deletions as come within the scope of the following claims and its equivalents.

SEQUENCE LISTING

(1) GENERAL INFORMATION:

(i) APPLICANT: Tolman, Richard L
Marburg, Stephen

(ii) TITLE OF INVENTION: PEPTIDE-POLYSACCHARIDE-PROTEIN CONJUGATE VACCINES

(iii) NUMBER OF SEQUENCES: 1

(iv) CORRESPONDENCE ADDRESS:
(A) ADDRESSEE: Merck & Co., Inc.
(B) STREET: P.O. Box 2000
(C) CITY: Rahway
(D) STATE: New Jersey
(E) COUNTRY: USA
(F) ZIP: 07065

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:

(A) NAME: Pfeiffer, Hesna J

(B) REGISTRATION NUMBER: 22640

(C) REFERENCE/DOCKET NUMBER: 18068IA

(ix) TELECOMMUNICATION INFORMATION:

(A) TELEPHONE: 908-594-4251

(B) TELEFAX: 908-594-4720

(2) INFORMATION FOR SEQ ID NO:1:

(i) SEQUENCE CHARACTERISTICS:

(A) LENGTH: 26 amino acids

(B) TYPE: amino acid

(D) TOPOLOGY: both

(ii) MOLECULE TYPE: peptide

(ix) FEATURE:

(A) NAME/KEY: Modified-site

(B) LOCATION: 1

(D) OTHER INFORMATION: /label= Nle

/note= "norleucine"

(ix) FEATURE:

(A) NAME/KEY: Disulfide-bond

(B) LOCATION: 2..26

(xi) SEQUENCE DESCRIPTION: SEQ ID NO:i:

Leu Cys Tyr Asn Lys Arg Lys Arg Ile His Ile Gly Pro Gly Arg Ala
1 5 10 15

Phe Tyr Thr Thr Lys Asn Ile Ile Gly Cys
20 25

## Claims

1. A conjugate comprising an HIV principle neutralizing determinant (PND) peptide, or a peptide (PEP) that is immunologically equivalent therewith, covalently coupled to an immunogenic protein or protein complex (PRO) through an anionic polysaccharide (PSA) linker.

2. The conjugate of Claim 1 wherein the immunogenic protein is the outer membrane protein complex (OMPC) of Neisseria meningititis b and the PND peptide has a linear structure, a disulfide bonded cyclic structure, an amide bonded cyclic structure, or a thioether bonded cyclic structure.

3. The conjugate of Claim 2 wherein the PND peptide is comprised of the sequence -GlyProGlyArg-, -GlyProGlyLys-, -GlyProGlyVal-, -GlyProGlyGln-, -GlyLeuGlyGln-, -GlyProGlyArgAlaPhe-, -HisIleGlyProGlyArgAlaPhe-, or -GlnArgGlyProGlyArgAlaPhe-.

4. The conjugate of Claim 3 wherein the PND peptide has a core or loop amino acid structure:
$-x-X_n-X_1-X_2-GPGR-X_3-X_4-X_m-y-$
or pharmaceutically acceptable salts thereof, wherein:
-GPGR- is the tetramer -GlyProGlyArg;
$X_1$ is:
a) serine,
b) proline,
c) arginine,
d) histidine,
e) glutamine, or
f) threonine;
$X_2$ is:
a) isoleucine,
b) arginine,
c) valine, or
d) methionine;
$X_n$ is either a bond or a peptide of up to 15 amino acids;
$X_3$ is:
a) alanine,
b) arginine, or
c) valine;
$X_4$ is:
a) phenylalanine,
b) isoleucine,
c) valine, or
d) leucine;
$X_m$ is bond or a peptide of up to 15 amino acids; and
-x- and -y- are joined to each other through a covalent structure, to form a cyclic peptide, which is covalently bound to PRO through an anionic polysaccharide.

**5.** The conjugate of Claim 4 wherein the cyclic covalent structure between x and y is comprised of:
a) an amide bond between an amino group on one side of the loop amino acids and a carboxyl group on the other side of the loop amino acids,
b) a xylylene thioether or lower alkyl thioether bond between sulfhydryl containing amino acids on either side of the loop amino acids, or
c) a disulfide bond between sulfhydryl containing amino acids on either side of the loop amino acids.

**6.** A conjugate comprising the structure IV:

$$\left[\left[PEP-r\right]_{PF}-PSA-B\right]_{GF}-PRO \qquad \underline{IV}$$

or pharmaceutically-acceptable salts thereof, wherein:

| | |
|---|---|
| PEP | comprises covalently coupled HIV PND peptide, having either a linear or a cyclic structure; |
| PSA | comprises anionic polysaccharide; |
| PRO | comprises the outer membrane protein complex (OMPC) of Neisseria meningitidis b; |
| -r- | comprises a spacer forming a covalent linkage; |
| -B- | comprises a spacer forming a covalent linkage; |
| PF = | peptidylation factor, in the range of 0.001 to 10 milligrams PEP per milligram PSA; |
| GF = | glycosylation factor, in the range of 0.001 to 10 milligrams PSA per milligram PRO; |
| ELF= | epitopic loading factor in milligrams PEP per milligram PRO in the range 0.0001 to 0.5 mg PEP/mg PRO. |

**7.** The conjugate of Claim 6 wherein PEP is limited to:

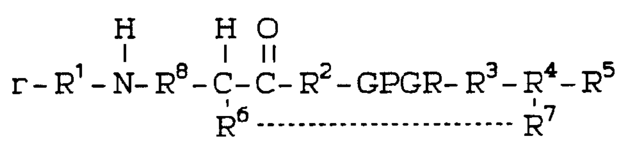

or pharmaceutically acceptable salts thereof, wherein:
r is the position of linkage between PEP and PSA;
$R^1$ is:
a) a bond, or
b) a peptide of 1 to 5 amino acids, optionally including a marker amino acid;
$R^2$ is :
a) either a bond or a peptide of up to 17 amino acids if $R^3$ is a peptide of at least 2 amino acids, or
b) a peptide of between 2 to 17 amino acids, if $R^3$ is a bond;
$R^3$ is:
a) either a bond or a peptide of up to 17 amino acids if $R^2$ is a peptide of at least 2 amino acids, or
b) a peptide of between 2 to 17 amino acids, if $R^2$ is a bond;
-GPGR- is the tetramer -GlyProGlyArg-;
$R^4$ is:
a) -NH-CH-CO- with $R^7$ bonded to the methine carbon if $R^7$ is $R^8$, or
b) a bond from $R^3$ to $R^7$ and $R^5$

if $R^7$ is:

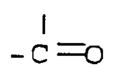 , or 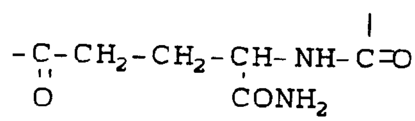 ;

$R^5$ is:
a) a peptide of one to five amino acids, optionally including a marker amino acid,
b) -OH,
c) -COOH,
d) $-CONH_2$,
e) $-NH_2$, or
f) -absent;
$R^6$ is:
a) an amino acid side chain, selected from the side chain of any of the common L or D amino acids, (see table of Definitions and Abbreviations), if the optional bond (--------) to $R^7$ is absent,
b) $-R^8$-S-S-, or $-R^8-S-R^8-R^9-R^8-S-$, if $R^7$ is $R^8$, or
c) $-R^8$-NH- if $R^7$ is

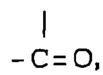
or
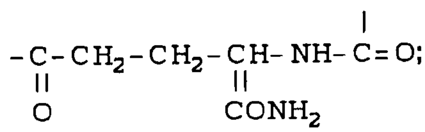

$R^7$ is:
a) $-R^8-$,
b)

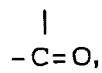
or

c)

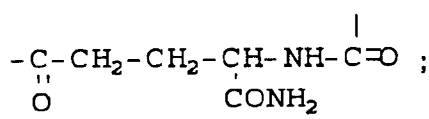

$R^8$ is a bond or lower alkyl of between one and eight carbons;
$R^9$ is:
a) $R^{10}$, or
b) xylylene;
$R^{10}$ is:
a) lower alkyl, or
b) $-CH_2OCH_2$; and
$-R^2$-GPGR-$R^3$- is the PEP core or loop amino acid structure.

**8.** The conjugate of Claim 7 wherein the PEP core amino acid structure $-R^2$-GPGR-$R^3$- is:
$-X_nX_1X_2$-GPGR-$X_3X_4X_m$-
wherein:
-GPGR- is the tetramer -GlyProGlyArg-;
$X_1$ is a constituent of $R^2$ selected from:
a) serine,
b) proline,
c) arginine,
d) histidine;
e) glutamine, or
f) threonine;
$X_2$ is a constituent of $R^2$ selected from:
a) isoleucine,

b) arginine,
c) valine, or
d) methionine;

$X_n$ is a constituent of $R^2$ and is either a bond or a peptide of up to 15 amino acids;

$X_3$ is a constituent of $R^3$ selected from:
a) alanine,
b) arginine, or
c) valine;

$X_4$ is a constituent of $R^3$ and is selected from:
a) phenylalanine,
b) isoleucine,
c) valine, or
d) leucine; and

$X_m$ is a constituent of $R^3$ and is a bond or a peptide of up to 15 amino acids.

**9.** The conjugate of Claim 8 wherein PEP is comprised of one or more of the peptides having the structure:

| Name | Structure |
|---|---|
| a) cPND1 | 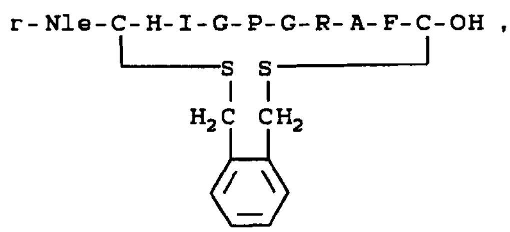 |
| b) cPND2 | 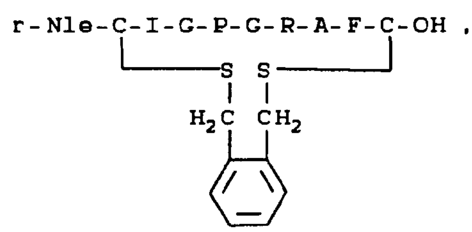 |
| c) cPND3 | 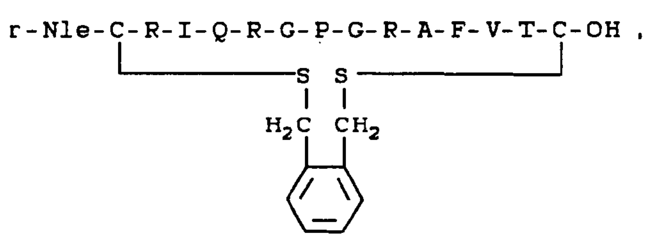 |
| d) cPND4 | 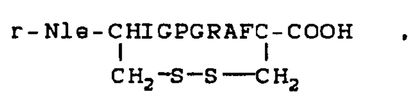 |

e) cPND8

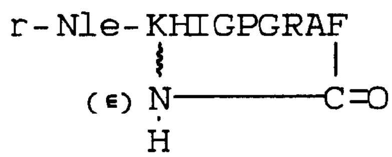

,

f) cPND9

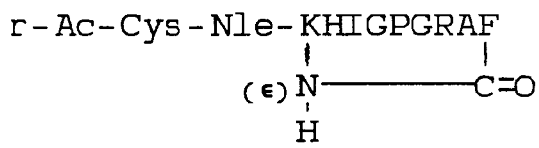

,

g) cPND11

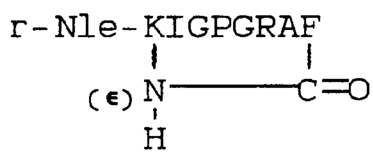

,

h) cPND12

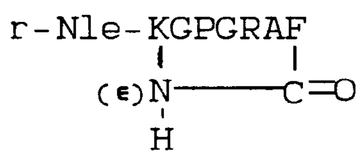

,

i) cPND13

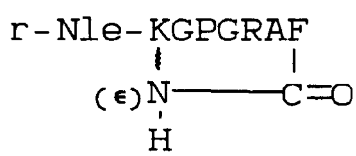

,

j) cPND14

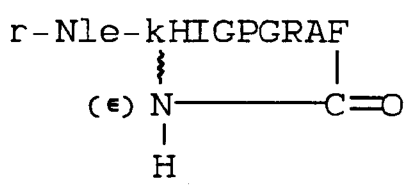

,

k) cPND15

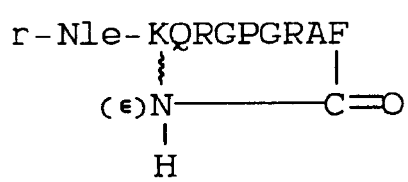

,

l) cPND16 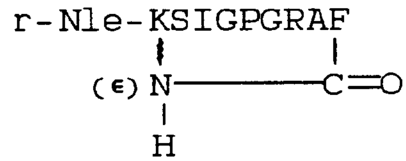 ,

m) cPND21 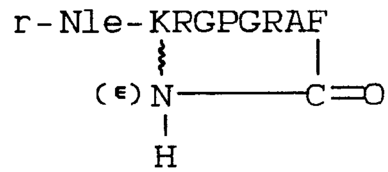 ,

n) cPND23 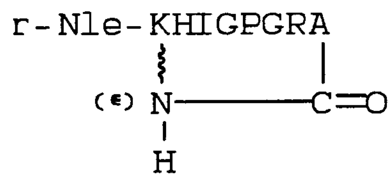 ,

o) cPND24 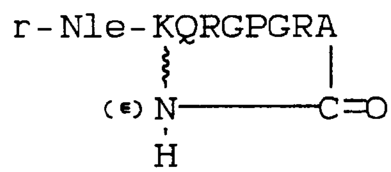 ,

p) cPND25 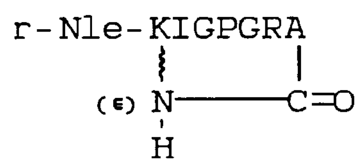 ,

q) cPND26 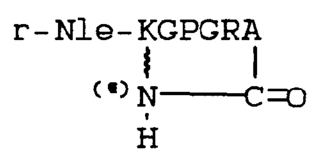 ,

r) cPND27 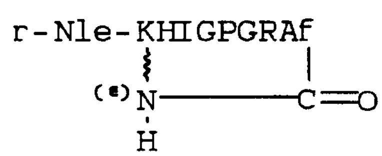 ,

s) cPND28 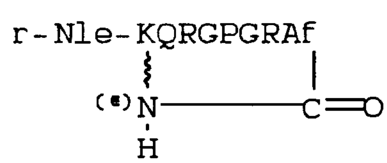 ,

t) cPND29 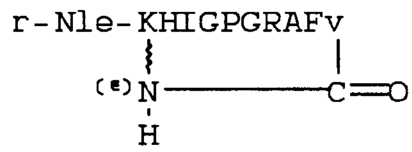 ,

u) cPND30 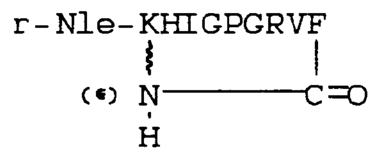 ,

v) cPND31 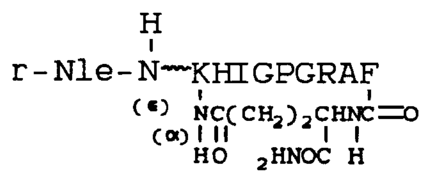 ,

w) cPND32 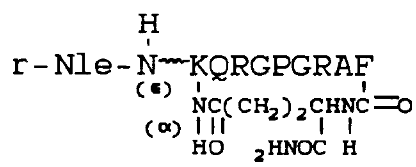 ,

x) PND142 r-YNKRKRIHIGPGRAFYTTKNIIGT,
y) PND-SC r-NNTTRSIHIGPGRAFYATGDIIGDI,
z) PND135 r-NNTRKSIRIQRGPGRAFVTIGKIGN,
aa) PND135-18 r-RIQRGPGRAFVTIGKIGN,
ab) PND135-12 r-RIQRGPGRAFVT,
ac) PND-MN8 r-HIGPGRAF,
ad) PND-MN6 r-GPGRAF,
ae) PND-LAV-1 r-IQRGPGRAF,
af) PND-SF2 r-IYIGPGRAF,
ag) PND-NY5 r-IAIGPGRTL,
ah) PND-CDC4 r-VTLGPGRVW,
ai) PND-RF r-ITKGPGRVI,
aj) PND-ELI r-TPIGLGQSL,
ak) PND-Z6 r-TPIGLGQAL,
al) PND-MAL r-IHFGPGQAL,
am) PND-Z3 r-IRIGPGKVF, or
an) cPND33:

H-Nle Cys Tyr Asn Lys Arg Lys Arg Ile His Ile Gly Pro
Gly Arg Ala Phe Tyr Thr Thr Lys Asn
$CH_2$ Ile Ile Gly Cys-OH
S S $CH_2$

;

wherein r is the position of linkage between the peptide and the anionic PSA of the conjugate, with the proviso that cPND33 may be linked through the amino terminal Nle or one of the internal Lys residues.

**10.** The conjugate of Claim 9 wherein the PEP is bonded to the polysaccharide through a linkage comprising an amide bond, a thioether bond, or a urethane bond.

**11.** The conjugate of Claim 10 wherein PSA is any of the anionic polysaccharides selected from among PRP, PnPs6A, PnPs6B, PnPs10A, PnPs11A, PnPs18C, PnPs19A, PnPs20, PnPs22F, and PnPs23F.

**12.** A peptide-polysaccharide-protein conjugate wherein the polysaccharide is comprised of PRP or PnPs6B and is covalently bound to peptide and protein through a spacer comprised of:
a) $-CONH(CH_2)_4NHCOCH_2SCH_2CH(NHCOCH_3)CO-$,
b) $-CONH(CH_2)_4NHCOCH_2SCH_2CH_2CH(NHCOCH_3)CO-$, or
c) $-CONH(CH_2)_2SCH_2CO-$;
the protein is comprised of the OMPC of Neisseria meningitidis b; and
the peptide is comprised of one or more of the peptides:

| Name | Structure |
|---|---|
| a) cPND1 | 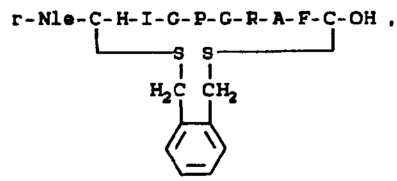 |
| b) cPND2 | 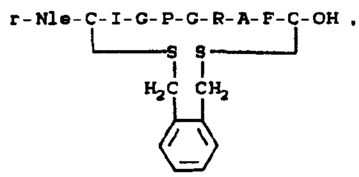 |
| c) cPND3 | 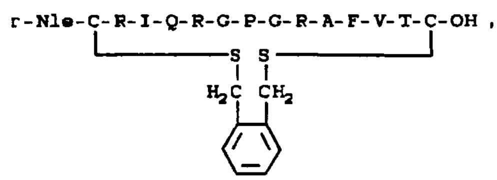 |
| d) cPND4 | 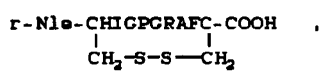 |
| e) cPND8 | 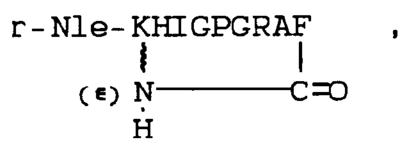 |
| f) cPND9 | 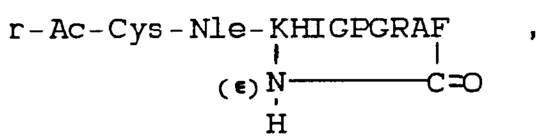 |
| g) cPND11 | 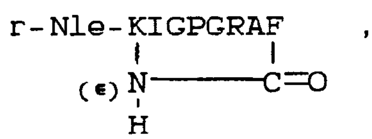 |

h) cPND12 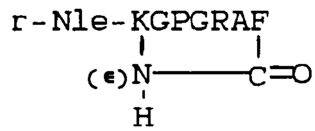 ,

i) cPND13 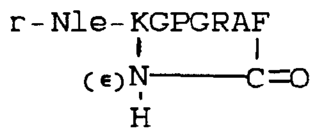 ,

j) cPND14 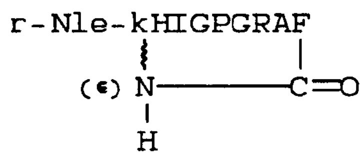 ,

k) cPND15 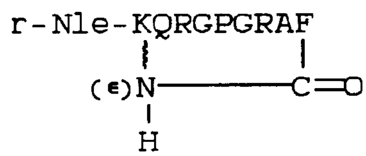 ,

l) cPND16 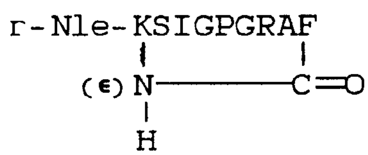 ,

m) cPND21 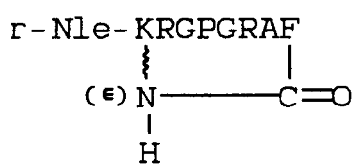 ,

n) cPND23 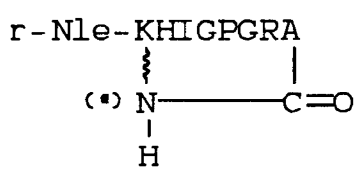 ,

o) cPND24 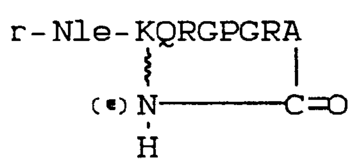 ,

p) cPND25 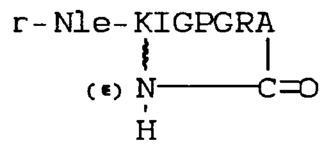 ,

q) cPND26 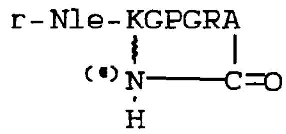 ,

r) cPND27 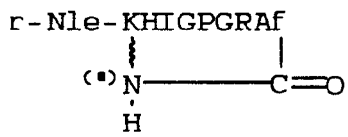 ,

s) cPND28 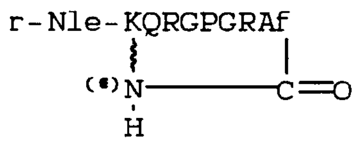 ,

t) cPND29 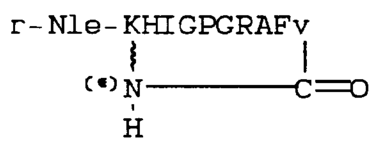 ,

u) cPND30 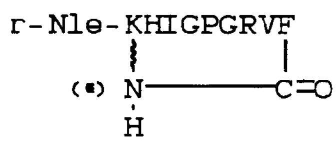 ,

v) cPND31 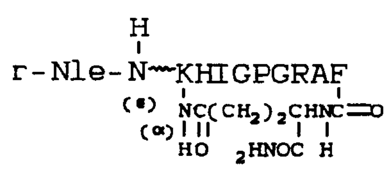 ,

w) cPND32 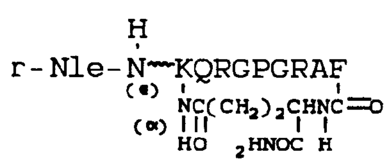 ,

x) PND142 r-YNKRKRIHIGPGRAFYTTKNIIGT,
y) PND-SC r-NNTTRSIHIGPGRAFYATGDIIGDI,
z) PND135 r-NNTRKSIRIQRGPGRAFVTIGKIGN,
aa) PND135-18 r-RIQRGPGRAFVTIGKIGN,
ab) PND135-12 r-RIQRGPGRAFVT,
ac) PND-MN8 r-HIGPGRAF,
ad) PND-MN6 r-GPGRAF,
ae) PND-LAV-1 r-IQRGPGRAF,
af) PND-SF2 r-IYIGPGRAF,
ag) PND-NY5 r-IAIGPGRTL,
ah) PND-CDC4 r-VTLGPGRVW,
ai) PND-RF r-ITKGPGRVI,
aj) PND-ELI r-TPIGLGQSL,
ak) PND-Z6 r-TPIGLGQAL,
al) PND-MAL r-IHFGPGQAL,
am) PND-Z3 r-IRIGPGKVF, or
an) cPND33:

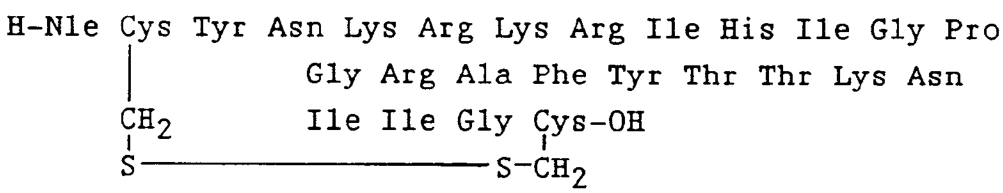

;

wherein r is the position of linkage between the peptide and PRP or PnPs6B, with the proviso that cPND33 may be linked through the amino terminal Nle or one of the internal Lys residues; and the conjugate has between 0.001 and 10 milligrams peptide per milligram PRP or PnPs6B, between 0.001 and 10 milligrams PRP or PnPs6B per milligram OMPC, and between 0.0001 to 0.5 milligrams peptide per milligram total protein.

**13.** A peptide-polysaccharide-protein conjugate having the structure:

a) 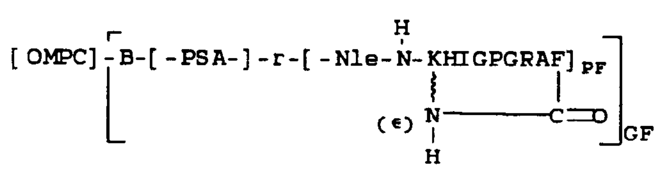 ,

b) 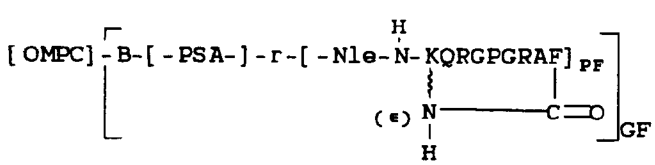 ,

c) 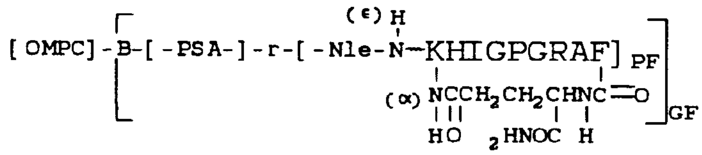 ,

d) 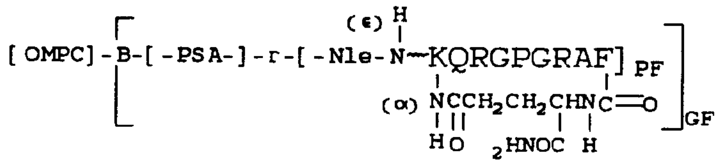 ,

e) 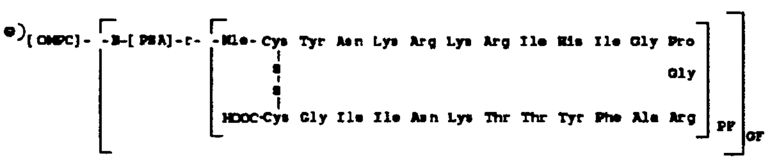 ,

f) 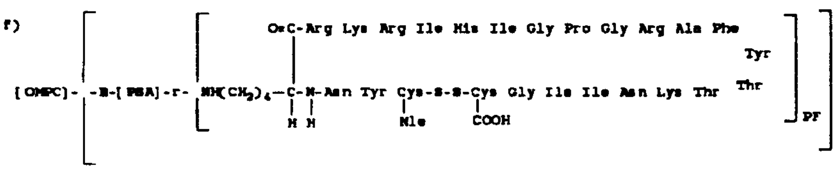 ,

g) 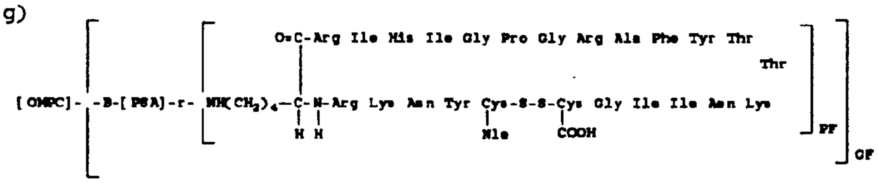 , or

h) 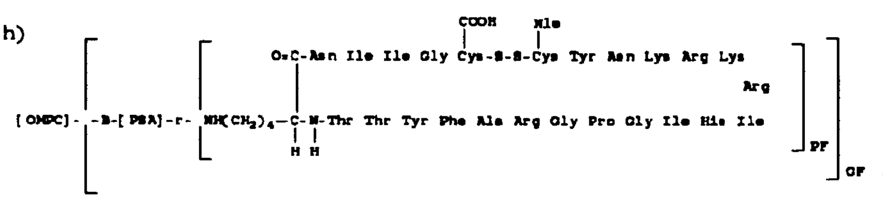 ,

wherein:

-r- and -B- are selected from:

a) $-CONH(CH_2)_4NHCOCH_2SCH_2CH(NHCOCH_3)CO-$,

b) $-CONH(CH_2)_4NHCOCH_2SCH_2CH_2CH(NHCOCH_3)CO-$, or

c) $-CONH(CH_2)_2SCH_2CO-$;

PSA is comprised of either PRP or PnPs6B; PF indicates a mass ratio of between 0.01 and 1 milligrams of peptide per milligram of PRP or PnPs6B;

and

GF indicates a mass ratio of between 0.01 and 0.33 milligrams of PRP or PnPs per milligram of OMPC.

**14.** A process for making the conjugate of Claim 1 which comprises the steps of (a) covalently conjugating PEP peptides to PSA; (b) conjugating the product of step (a) with an immunogenic protein PRO.

**15.** The process of Claim 14 which comprises the steps of (a-i) derivatizing PEP peptides with a thiolating reagent; (a-ii) reacting PSA with a bromoacetyl donor; (a-iii) reacting the product of step (a-i) with the product of step (a-ii); (b-i) derivatizing PRO with a thiolating reagent; (b-ii) reacting the product of step (b-i) with the product of step (a-iii); (b-iii) capping the product of step (b-ii) with N-acetylcysteamine to quench residual bromoacetyl moieteies on PSA; and (b-iv) isolating the conjugate free of reactants.

**16.** The process of Claim 14 which comprises the steps of (a-i) derivatizing PEP peptides with a bromoacetyl donor; (a-ii) preparing thiolated PSA by reacting PSA with a thiol donor; (a-iii) reducing the product of step (a-ii) to generate free sulfhydryls on PSA; (a-iv) reacting the product of step (a-iii) with the product of step (a-i); (b-i) derivatizing PRO with a bromoacetyl donor; (b-ii) reacting the product of step (b-i) with the product of step (a-iv); (b-iii) capping the product of step (b-ii) with N-ethyl maleimide to quench residual free sulfhydryls on PSA; and (b-iv) isolating the conjugate free of reactants.

**17.** The process of Claim 15 wherein the thiol donor is N-acetylhomocysteine thiolactone or cystamine, and the bromoacetyl donor is p-nitrophenyl bromoacetate, or N-(bromoacetyl)-6-aminocaproic acid p-nitrophenyl ester.

**18.** The process of Claim 16 wherein the thiol donor is N-acetylhomocysteine thiolactone or cystamine, and the bromoacetyl donor is p-nitrophenyl bromoacetate, or N-(bromoacetyl)-6-aminocaproic acid p-nitrophenyl ester.

**19.** A pharmaceutical composition, comprising an inert carrier and the conjugate of either Claim 1, Claim 6, Claim 12, or Claim 13.

**20.** A pharmaceutical composition comprising a cocktail of conjugates of either Claim 1, Claim 6, Claim 12 or Claim 13, wherein said cocktail is prepared by making different conjugates each with a different PND peptide or mixture of PND peptides, and then mixing these conjugates to form the composition.

**21.** A composition comprising a cocktail of conjugates of either Claim 1, Claim 6, Claim 12 or Claim 13 wherein the cocktail of conjugates is adsorbed to aluminum hydroxide gel in physiological saline.

**22.** The use of a conjugate as claimed in Claim 1, Claim 6, Claim 12 or Claim 13 for the preparation of a medicament for inducing anti-peptide, anti-HIV or HIV-neutralizing immune responses in a mammal.

**23.** The use of a conjugate as claimed in Claim 1, Claim 6, Claim 12 or Claim 13 for the preparation of a medicament for the treatment of AIDS, or infection by HIV.

**24.** Antiserum raised by inoculating a mammal with a composition comprising the conjugate as claimed in any of Claims 1, 6, 12 or 13.

**25.** The use of antiserum as claimed in Claim 24 for the preparation of a medicament for passively protecting a human against infection by HIV, or protecting a human against proliferation of HIV post-infection, or treating a human afflicted with AIDS.